# EUROPEAN PATENT APPLICATION

(11) **EP 4 792 747 A1**
(43) Date of publication of application: **19.08.2026**
(21) Application number: 24911130.3
(22) Date of filing: 24.12.2024
(51) Int. Cl.: A61B 6/04, A61B 5/055, A61D 3/00

(54) **ANIMAL TRAY, MULTI-IMAGED-ANIMAL FIXING APPARATUS, AND ANIMAL IMAGING SYSTEM AND METHOD**

(30) Priority: 26.12.2023 CN 202311813876; 11.05.2024 CN 202410586308; 11.05.2024 CN 202421025620 U; 02.09.2024 CN 202411226565; 02.09.2024 CN 202411223932; 02.09.2024 CN 202411223881
(71) Applicant: Wuhan United Imaging Life Science Instrument Co., Ltd, Wuhan, Hubei 430206 (CN)
(72) Inventor: WANG, Sheng, Wuhan, Hubei 430206 (CN); ZHU, Longwei, Wuhan, Hubei 430206 (CN); WANG, Yanjun, Wuhan, Hubei 430206 (CN); HUANG, Sicen, Wuhan, Hubei 430206 (CN); ZHAI, Renkuan, Wuhan, Hubei 430206 (CN); XING, Chao, Wuhan, Hubei 430206 (CN)
(74) Representative: Wang, Bo
(86) International application number: PCT/CN2024/141923
(87) International publication number: WO 2025/140205

(57) **Abstract**

An animal tray (200), a multi-imaging-animal fixation device (110), a system (100) and a method for animal imaging are provided. The method includes: scanning an imaging animal using an imaging device (150) to acquire scan data of the imaging animal; and reconstructing a target medical image of the imaging animal based on the scan data. During the scan, the imaging animal is placed on a tray (200) in a preset positioning posture and is placed into the imaging device (150) via the tray (200). The tray (200) includes body part fixation portions (201) disposed at fixed positions on the tray (200) and configured to maintain the preset positioning posture of the imaging animal.

## Description

### CROSS-REFERENCE TO RELATED APPLICATIONS

This application claims priority to the Chinese Patent Application No. 202411226565.1, 202411223932.2, and 202411223881.3, all filed on September 2, 2024, claims priority to the Chinese Patent Application No. 202410586308.2 and 202421025620.6, both filed on May 11, 2024, and claims priority to the Chinese Patent Application No. 202311813876.3, filed on December 26, 2023, the contents of each of which are hereby incorporated by reference.

### TECHNICAL FIELD

The present disclosure relates to the field of animal imaging, and in particular, to an animal tray, a multi-imaging-animal fixation device, and a system and a method for animal imaging.

### BACKGROUND

Animal experiments refer to scientific research conducted using animals (e.g., mice) to acquire new knowledge in biology, medicine, or to solve specific problems. In some animal experiments, it is necessary to perform scans on the imaging animals. To ensure image quality (e.g., to reduce motion artifacts), the imaging animals need to be fixed. However, existing imaging animal fixation solutions fail to achieve standardized positioning of the imaging animals and exhibit low placement efficiency. For example, laboratory personnel may use adhesive tape to fix a mouse in a random posture on a flat surface. Furthermore, existing animal imaging methods generally directly fix the imaging animals within an animal chamber, placing high demands on the chamber itself and failing to satisfy the requirements of animal experiments. Existing animal chambers are essentially custom products based on specific scanning equipment and cannot satisfy diversified experimental needs. The structure of the animal chamber and its animal fixation devices are typically provided by the manufacturer, making them unable to adapt to different experimental requirements of different users, resulting in low experimental efficiency.

Therefore, an animal tray, a multi-imaging-animal fixation device, and a system and a method for animal imaging are provided to improve the efficiency of animal experiments.

### SUMMARY

One or more embodiments of the present disclosure provide a tray for placing an imaging animal. The tray includes: body part fixation portions disposed at fixed positions on the tray and configured to fix body parts of the imaging animal placed on the tray, thereby maintaining a positioning posture of the imaging animal; and one or more marker accommodation portions configured to accommodate a marker. The marker is detectable in at least one imaging modality.

In some embodiments, the body part fixation portions include: a head fixation portion configured to fix the head of the imaging animal; a forelimb fixation portion configured to fix the forelimbs of the imaging animal; and a hindlimb fixation portion configured to fix the hindlimbs of the imaging animal.

In some embodiments, each of the forelimb fixation portion and the hindlimb fixation portion includes a groove structure or a hole structure.

In some embodiments, the head fixation portion includes a bite bar for the teeth of the imaging animal to bite, thereby fixing the teeth of the imaging animal.

In some embodiments, the head fixation portion further includes a head cap configured to accommodate the head of the imaging animal.

In some embodiments, the body part fixation portions further include a tail fixation portion configured to fix the tail of the imaging animal.

In some embodiments, the tail fixation portion is a groove structure.

In some embodiments, the forelimb fixation portion includes a first hole structure that penetrates the tray and is configured for the forelimb of the imaging animal to pass through; the hindlimb fixation portion includes a second hole structure that penetrates the tray and is configured for the hindlimb of the imaging animal to pass through; and the first hole structure and the second hole structure are waist-shaped holes.

In some embodiments, the imaging animal has a plurality of forelimbs, and the first hole structure includes a first hole structure corresponding to each of the plurality of forelimbs; the imaging animal has a plurality of hindlimbs, and the second hole structure includes a second hole structure corresponding to each of the plurality of hindlimbs.

In some embodiments, the one or more marker accommodation portions include a first marker accommodation portion and a second marker accommodation portion. The first marker accommodation portion is disposed at a first position on the tray proximate to the forelimb fixation portion, and the second marker accommodation portion is disposed at a second position on the tray proximate to the hindlimb fixation portion.

In some embodiments, each marker accommodation portion includes a first accommodation channel, a second accommodation channel, and a marker accommodation cavity, the first accommodation channel is configured for injecting the marker, the second accommodation channel is configured for expelling gas during injection of the marker, and the marker accommodation cavity is respectively in communication with the first accommodation channel and the second accommodation channel and is configured to accommodate the marker.

In some embodiments, the first accommodation channel and the second accommodation channel are hollow cylinders, the marker accommodation cavity is spherical, and a center of the marker accommodation cavity is located on a central axis of the first accommodation channel and the second accommodation channel.

In some embodiments, a diameter of the first accommodation channel is greater than a diameter of the second accommodation channel.

In some embodiments, the tray is provided with one or more fixing structures, and each marker accommodation portion is detachably mounted to the tray via one of the one or more fixing structures.

In some embodiments, each fixing structure includes a clamping ring structure, the clamping ring structure includes elastic clamping ring arms configured to secure the corresponding marker accommodation portion.

In some embodiments, the marker is detectable in at least one first imaging modality, the marker accommodation portion is detectable in at least one second imaging modality, the at least one first imaging modality includes at least one of positron emission imaging, single-photon emission imaging, or magnetic resonance imaging (MRI), and the at least one second imaging modality includes X-ray imaging.

In some embodiments, at least one marker accommodation portion is configured to accommodate a marker detectable in an MRI modality, and the at least one marker accommodation portion is integrally formed with the tray.

In some embodiments, the marker is detectable in a plurality of imaging modalities.

In some embodiments, a first end portion of the tray for placing the head of the imaging animal is provided with an anesthesia interface configured to deliver anesthetic gas to the imaging animal.

In some embodiments, the anesthesia interface includes a first anesthesia channel and a second anesthesia channel, the first anesthesia channel includes a first end and a second end, the second anesthesia channel includes a third end and a fourth end, the first end is an inlet for the anesthetic gas, the third end is an outlet for the anesthetic gas and is located near a placement position of the nose of the imaging animal, the second end is in communication with the fourth end, and at a communication point, a preset angle is formed between the second end and the fourth end.

In some embodiments, the tray further includes: a physiological signal acquisition device configured to acquire a physiological signal of the imaging animal.

In some embodiments, the tray further includes: a receiving coil, the receiving coil is embedded in a bottom of the tray and configured to acquire MR data during an MR scan of the imaging animal.

In some embodiments, the receiving coil is disposed around an airbag of a respiration signal acquisition device.

One or more embodiments of the present disclosure provide a multi-imaging-animal fixation device. The multi-imaging-animal fixation device includes: a plurality of trays as described above and a support portion, the support portion being configured to support the plurality of trays, wherein: each of the plurality of trays includes a first end portion and a second end portion, the first end portion is configured to place the head of the imaging animal, and the second end portion is disposed opposite to the first end portion; the support portion includes a first portion and a second portion disposed opposite to each other, the first portion is configured to support the first end portions of the plurality of trays, and the second portion is configured to support the second end portions of the plurality of trays; and the support portion further includes a third portion configured to connect the first portion and the second portion.

In some embodiments, a count of the plurality of trays is in a range of 2 to 4.

In some embodiments, the third portion includes: a support beam, suspended between and connected to the first portion and the second portion; and a support base, disposed at the bottom of the multi-imaging-animal fixation device and configured to support the first portion, the second portion, and the support beam.

In some embodiments, the third portion is provided with a hollowed-out portion.

In some embodiments, the first portion is provided with an indication mark for indicating a placement position for the first end portions of the plurality of trays.

In some embodiments, the first end portion of each of the plurality of trays is further provided with an anesthesia interface configured to deliver an anesthetic gas to the imaging animal, and the anesthesia interface is provided with an inwardly recessed snap-fit structure; the first portion of the support portion is provided with a plurality of engagement structures corresponding to the plurality of trays, and each of the plurality of engagement structures includes an outwardly protruding snap-fit boss; and the snap-fit boss of each of the plurality of engagement structures is configured to engage with the snap-fit structure of the corresponding tray, such that the anesthesia interface and the engagement structure are snap-fit engaged.

In some embodiments, the plurality of trays in the multi-imaging-animal fixation device are arranged in at least two layers, and the trays in different layers are connected via the support portion.

In some embodiments, the first portion, the second portion, and the third portion are independent components capable of being assembled to form the support portion.

In some embodiments, the support portion further includes: a connector disposed on the first portion and/or the second portion, configured to connect the multi-imaging-animal fixation device and another imaging animal fixation device.

One or more embodiments of the present disclosure provide an animal chamber, comprising: a chamber body; and a tray as described in any one of the above embodiments or a multi-imaging-animal fixation device as described in any one of the above embodiments, wherein the tray or the multi-imaging-animal fixation device is detachably placed within the chamber body.

In some embodiments, the chamber body is provided with an indication mark for indicating a center of an MR coil, for assisting in placement and movement of the tray within the chamber body to align an imaging site of the imaging animal with the center of the MR coil.

In some embodiments, the tray is provided with a scale indicator device, and the scale indicator device and the indication mark cooperate to assist in placement and movement of the tray within the animal chamber.

In some embodiments, the chamber body is provided with a slide rail extending along a longitudinal axis thereof; the tray is provided with a slider; and the tray and the chamber body are slidably connected via the slide rail and the slider, such that the tray is movable within the chamber body along the longitudinal axis.

One or more embodiments of the present disclosure provide an animal imaging system, comprising: an imaging device having a scanning cavity; and an animal chamber as described above, wherein: the animal chamber is movable into the scanning cavity; and the imaging device is configured to scan the imaging animal within the animal chamber.

In some embodiments, the imaging device includes at least one of a computed tomography (CT) device, an MRI device, a positron emission computed tomography (PET) device, or a single-photon emission computed tomography (SPECT) device, or a multi-modality imaging device formed by a combination of two or more devices selected from the CT device, the MRI device, the PET device, and the SPECT device.

One or more embodiments of the present disclosure provide a method for scanning an imaging animal. The method comprises: scanning the imaging animal using an imaging device to acquire scan data of the imaging animal; and reconstructing a target medical image of the imaging animal based on the scan data, wherein: during the scan, the imaging animal is placed on a tray in a preset positioning posture and is placed into the imaging device via the tray; and the tray includes body part fixation portions disposed at fixed positions on the tray and configured to maintain the preset positioning posture of the imaging animal.

In some embodiments, the tray includes an anesthesia interface disposed at a first end portion of the tray, the first end portion is configured for placing the head of the imaging animal; and during the scan, anesthetic gas is delivered to the imaging animal through the anesthesia interface to keep the imaging animal in an anesthetized state.

In some embodiments, the imaging animal includes a plurality of imaging animals, each of the plurality of imaging animals is placed on one tray; a plurality of trays corresponding to the plurality of imaging animals are placed on a support portion of a multi-imaging-animal fixation device; and the plurality of imaging animals are placed into the imaging device via the multi-imaging-animal fixation device.

In some embodiments, the scan is a static emission computed tomography (ECT) scan, and before the scan, the method further comprises: injecting a tracer into the plurality of imaging animals; after a preset time period following the tracer injection, placing the plurality of imaging animals in an induction box and performing a pre-anesthesia treatment on the plurality of imaging animals; and placing the plurality of imaging animals after the pre-anesthesia treatment on the plurality of trays in the preset positioning posture.

In some embodiments, the scan is a dynamic ECT scan, and before the scan, the method further comprises: placing the plurality of imaging animals in an induction box and performing a pre-anesthesia treatment on each of the plurality of imaging animals; fixing an indwelling needle on each of the plurality of imaging animals after the pre-anesthesia treatment; placing the plurality of imaging animals after the pre-anesthesia treatment and the fixation of the indwelling needles on the plurality of trays in the preset positioning posture; and injecting a tracer into each of the plurality of imaging animals using the indwelling needles.

In some embodiments, the imaging device includes a first imaging device and a second imaging device, and the scan data includes first scan data acquired by the first imaging device and second scan data acquired by the second imaging device; the reconstructing a target medical image of the imaging animal based on the scan data includes: reconstructing, based on the first scan data, a first medical image of the imaging animal; reconstructing, based on the second scan data, a second medical image of the imaging animal; and performing registration and fusion on the first medical image and the second medical image to generate the target medical image. The tray is provided with one or more marker accommodation portions configured to accommodate a marker that is detectable in the first medical image and the second medical image, and the registration and fusion are performed based on the marker in the first medical image and the second medical image.

In some embodiments, one of the first imaging device and the second imaging device is an ECT device, and the other is an MRI device.

In some embodiments, the imaging device is an MRI device, and the scan is an enhanced MR scan; before the scan, the method further comprises: placing the imaging animal in an induction box and performing a pre-anesthesia treatment and fixing an indwelling needle on the imaging animal; placing the imaging animal after the pre-anesthesia treatment and the fixation of the indwelling needle on the tray in the preset positioning posture, and placing the imaging animal into the MRI device via the tray; and performing contrast agent injection on the imaging animal via the indwelling needle.

In some embodiments, the imaging device is an MRI device, and the scan is an enhanced MR scan; the method further includes: before the scan: placing the imaging animal in an induction box and performing a pre-anesthesia treatment and fixing an indwelling needle on the imaging animal; placing the imaging animal after the pre-anesthesia treatment and the fixation of the indwelling needle on the tray in the preset positioning posture, and placing the imaging animal into the MRI device via the tray; and connecting the indwelling needle to an external injection device; and during the scan, performing contrast agent injection on the imaging animal via the external injection device and the indwelling needle.

In some embodiments, the method further includes: before performing the contrast agent injection on the imaging animal, obtaining an initial MR image by scanning the imaging animal using the MRI device; and performing comparative analysis on the initial MR image and an enhanced MR image.

In some embodiments, the imaging device is an MRI device; the scanning the imaging animal using an imaging device to acquire scan data of the imaging animal includes: placing the tray at an initial position within the animal chamber, and placing the animal chamber in an imaging region of the MRI device, such that a first part of the imaging animal is located within the imaging region; performing a first scan on the first part of the imaging animal using the MRI device to acquire first MR data; moving a position of the tray within the animal chamber, and re-placing the animal chamber into the imaging region, such that a second part of the imaging animal is located within the imaging region, where the first part and the second part have a partial overlapping region; and performing a second scan on the second part of the imaging animal using the MRI device to acquire second MR data.

In some embodiments, reconstructing a target medical image of the imaging animal based on the scan data includes: reconstructing a first MR image based on the first MR data; reconstructing a second MR image based on the second MR data; and stitching the first MR image and the second MR image to obtain a stitched MR image as the target medical image.

In some embodiments, the tray is provided with one or more marker accommodation portions, each marker accommodation portion being for accommodating a marker detectable in an MRI mode; at least one marker accommodation portion of the one or more marker accommodation portions is located within the imaging region during both the first scan and the second scan; and the stitching is performed based on the at least one marker accommodation portion in the first MR image and the at least one marker accommodation portion in the second MR image.

In some embodiments, the method further includes: before placing the animal chamber in the imaging region, installing an MR coil on an exterior of the animal chamber; before moving the position of the tray within the animal chamber, moving the animal chamber out of the imaging region and removing the MR coil; and before re-placing the animal chamber into the imaging region, re-installing the MR coil on the exterior of the animal chamber.

In some embodiments, the tray includes a physiological signal acquisition device configured to acquire a physiological signal of the imaging animal; reconstructing a target medical image of the imaging animal based on the scan data includes: performing gated reconstruction on the scan data based on the physiological signal to generate the target medical image.

In some embodiments, the imaging device is an MRI device; each tray includes a receiving coil embedded in a bottom of the tray; the scan data includes MR data acquired by the receiving coil of each tray from the imaging animal placed on that tray; and the reconstructing a target medical image of the imaging animal based on the scan data includes: for each tray, generating the target medical image of the imaging animal placed on the tray based on the MR data acquired by the receiving coil of the tray.

### BRIEF DESCRIPTION OF THE DRAWINGS

The present disclosure will be further illustrated by way of exemplary embodiments, which will be described in detail through the accompanying drawings. These embodiments are not limiting, and in these embodiments, the same numbering denotes the same structure.
FIG. 1 is a schematic diagram illustrating an exemplary animal imaging system according to some embodiments of the present disclosure;
FIG. 2 is a schematic diagram illustrating an exemplary animal tray according to some embodiments of the present disclosure;
FIG. 3 is a schematic diagram illustrating another exemplary animal tray according to some embodiments of the present disclosure;
FIG. 4A and FIG. 4B are schematic diagrams illustrating an exemplary marker accommodation portion according to some embodiments of the present disclosure;
FIG. 5 is an enlarged schematic diagram illustrating Region I in FIG. 3 according to some embodiments of the present disclosure;
FIG. 6 is a schematic diagram illustrating an exemplary anesthesia interface according to some embodiments of the present disclosure;
FIG. 7 is a schematic diagram illustrating another exemplary animal tray according to some embodiments of the present disclosure;
FIG. 8 is a schematic diagram illustrating an exemplary multi-imaging-animal fixation device according to some embodiments of the present disclosure;
FIG. 9A is a schematic diagram illustrating an exemplary support portion of a multi-imaging-animal fixation device according to some embodiments of the present disclosure;
FIG. 9B is an enlarged schematic diagram illustrating Region II in FIG. 9A according to some embodiments of the present disclosure;
FIG. 9C is a schematic diagram illustrating a disassembled support portion according to some embodiments of the present disclosure;
FIG. 10A is a schematic diagram illustrating another exemplary multi-imaging-animal fixation device according to some embodiments of the present disclosure;
FIG. 10B is an enlarged schematic diagram illustrating Region III in FIG. 10A according to some embodiments of the present disclosure;
FIG. 11 is a cross-sectional schematic diagram illustrating an exemplary multi-imaging-animal fixation device according to some embodiments of the present disclosure;
FIG. 12 is a schematic diagram illustrating an exemplary animal chamber according to some embodiments of the present disclosure;
FIG. 13 is a schematic diagram illustrating another exemplary animal chamber according to some embodiments of the present disclosure;
FIG. 14 is a schematic diagram illustrating yet another exemplary animal chamber according to some embodiments of the present disclosure;
FIG. 15 is a flowchart illustrating an exemplary process for scanning an imaging animal according to some embodiments of the present disclosure;
FIG. 16 is a flowchart illustrating an exemplary process for animal emission computed tomography (ECT) scanning according to some embodiments of the present disclosure;
FIG. 17 is a flowchart illustrating an exemplary static ECT scan process according to some embodiments of the present disclosure;
FIG. 18 is a flowchart illustrating an exemplary dynamic ECT scan process according to some embodiments of the present disclosure;
FIG. 19 is an image illustrating an exemplary result of registration and fusion based on first modality scan data and second modality scan data according to some embodiments of the present disclosure;
FIG. 20 is a flowchart illustrating an exemplary process for generating a target medical image according to some embodiments of the present disclosure;
FIG. 21 is a flowchart illustrating an exemplary PET scanning and MR scanning process according to some embodiments of the present disclosure;
FIG. 22 is an image illustrating an exemplary fusion result of a PET scan image and an MR scan image according to some embodiments of the present disclosure;
FIG. 23A is a flowchart illustrating an exemplary process for an animal enhanced MR scan according to some embodiments of the present disclosure;
FIG. 23B is a flowchart illustrating an exemplary process for performing an MR scan and an enhanced MR scan on an imaging animal according to some embodiments of the present disclosure;
FIG. 24A is a flowchart illustrating another exemplary process for an animal enhanced MR scan according to some embodiments of the present disclosure;
FIG. 24B is a flowchart illustrating another exemplary process for performing an MR scan and an enhanced MR scan on an imaging animal according to some embodiments of the present disclosure;
FIG. 25A is a schematic diagram illustrating an enhanced MR image of a brain tumor of an imaging animal according to some embodiments of the present disclosure;
FIG. 25B is a schematic diagram illustrating a gadolinium concentration map in the intercellular space of an imaging animal according to some embodiments of the present disclosure;
FIG. 25C is a schematic diagram illustrating a gadolinium volume transfer rate map of an imaging animal according to some embodiments of the present disclosure;
FIG. 26 is a schematic diagram illustrating exemplary enhanced MR images at different time periods according to some embodiments of the present disclosure;
FIG. 27 is a flowchart illustrating an exemplary process of animal MRI according to some embodiments of the present disclosure;
FIG. 28 is a schematic diagram illustrating an exemplary stitched MR image of a mouse, according to some embodiments of the present disclosure; and
FIG. 29 is a flowchart illustrating another exemplary process of animal MRI according to some embodiments of the present disclosure.

### DETAILED DESCRIPTION

In order to illustrate the technical solutions of the embodiments of the present disclosure more clearly, the accompanying drawings required for describing the embodiments are briefly introduced below. Obviously, drawings described below are only some examples or embodiments of the present disclosure. Those skilled in the art, without further creative efforts, may apply the present disclosure to other similar scenarios according to these drawings. It should be understood that the purposes of these illustrated embodiments are only provided to those skilled in the art to practice the application, and not intended to limit the scope of the present disclosure. Unless obviously obtained from the context or the context illustrates otherwise, the same numeral in the drawings refers to the same structure or operation.

It will be understood that the term "system," "device," "unit," and/or "module" used herein are one method to distinguish different components, elements, parts, sections or assemblies of different levels in ascending order. However, the terms may be displaced by another expression if they achieve the same purpose.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in the present disclosure, specify the presence of stated operations and/or elements, but do not preclude the presence or addition of one or more other operations and/or elements thereof.

The flowcharts used in the present disclosure illustrate operations that systems implement according to some embodiments of the present disclosure. It is to be expressly understood, the operations of the flowcharts may be implemented not in order. Conversely, the operations may be implemented in an inverted order, or simultaneously. Moreover, one or more other operations may be added to the flowcharts. One or more operations may be removed from the flowcharts.

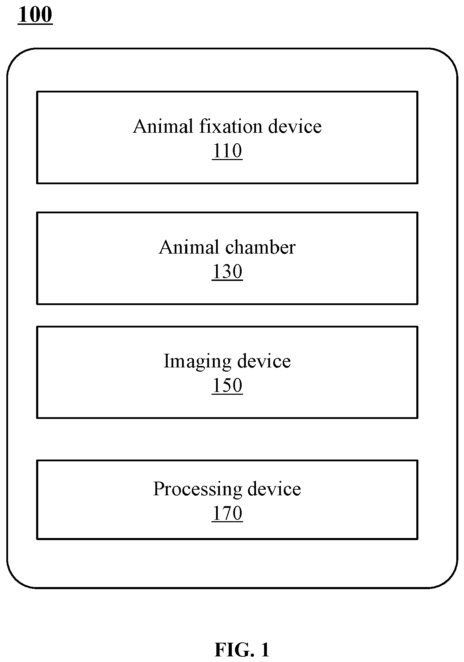

FIG. 1 is a schematic diagram illustrating an exemplary animal imaging system according to some embodiments of the present disclosure. As shown in FIG. 1, an animal imaging system 100 includes an animal fixation device 110, an animal chamber 130, an imaging device 150, and a processing device 170.

The animal fixation device 110 may be configured to fix an imaging animal. The imaging animal may include various small mammals such as a guinea pig, a hamster, a bamboo rat, a ferret, a rabbit, or the like. In some embodiments of the present disclosure, the imaging animal will be described primarily using rodents (e.g., mice or rats) as examples.

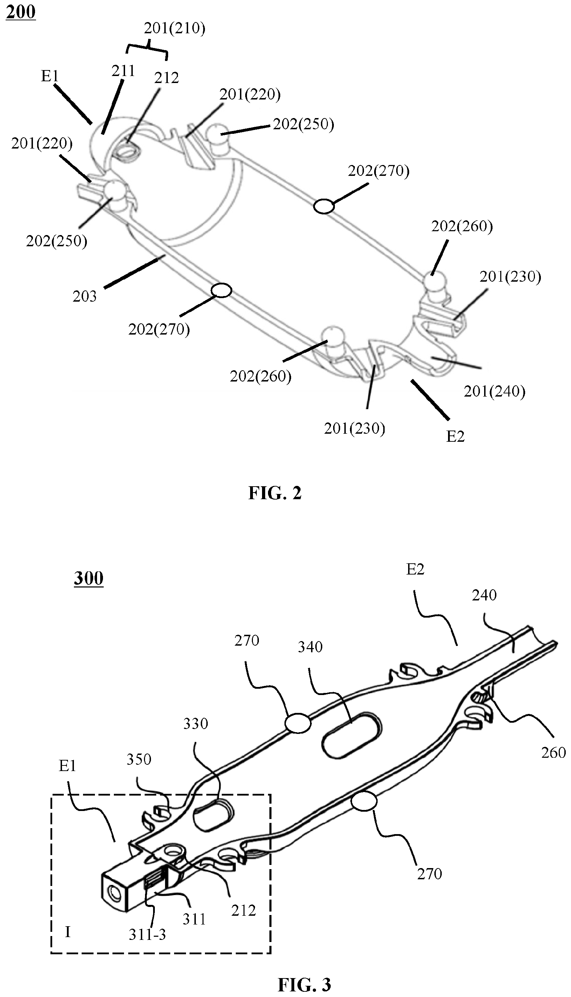

In some embodiments, the animal fixation device 110 includes an animal tray (also referred to as a tray) for placing a single imaging animal, which may also be referred to as a single-imaging-animal fixation device. More descriptions regarding the animal tray may be found in FIGs. 2 to 7 and the related descriptions thereof.

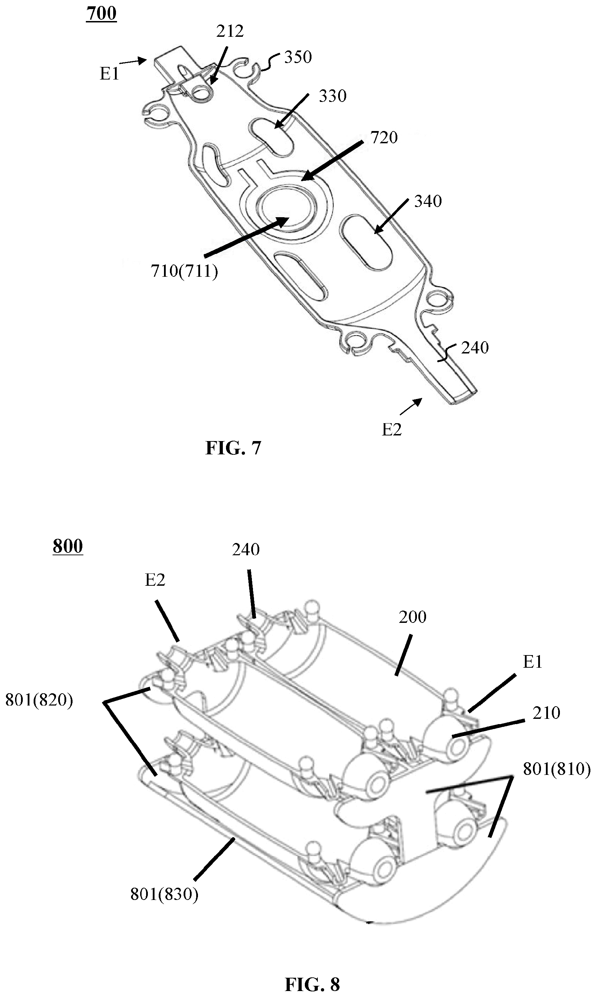

In some embodiments, the animal fixation device 110 includes a multi-imaging-animal fixation device for simultaneously placing a plurality of imaging animals. The multi-imaging-animal fixation device includes a plurality of animal trays and a support portion configured to support the plurality of animal trays. More descriptions regarding the multi-imaging-animal fixation device may be found in FIGs. 8-11 and the related descriptions thereof.

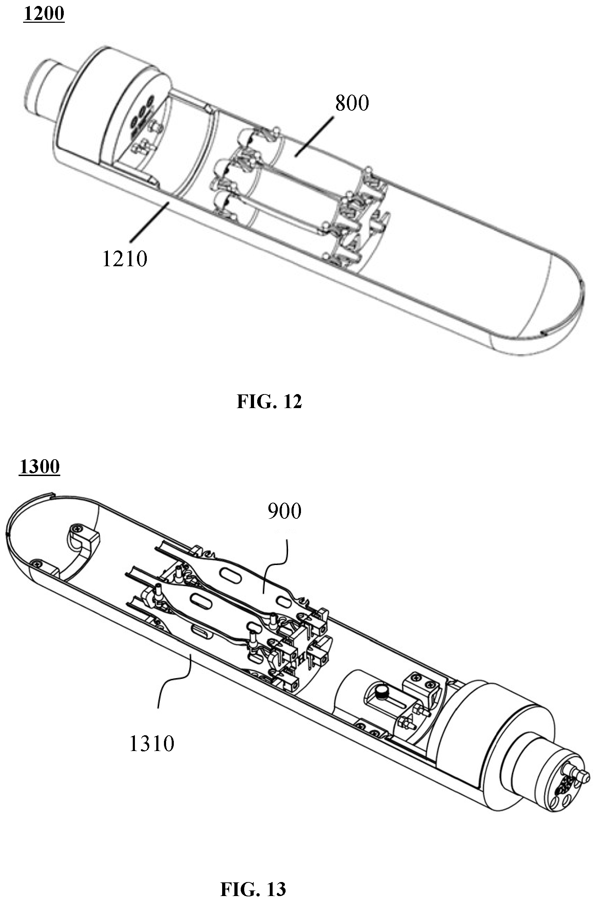

The animal chamber 130 may be configured to accommodate one or more animal fixation devices 110 (such as an animal tray, a multi-imaging-animal fixation device). In some embodiments, the animal fixation device 110 is detachably connected to the chamber body of the animal chamber 130. Merely by way of example, the multi-imaging-animal fixation device may be detachably fixed to an inner wall of the chamber body through snap-fit connections, straps, adhesive tape, or the like. Alternatively, the multi-imaging-animal fixation device may be directly placed against the inner wall of the chamber body. More descriptions regarding the animal chamber may be found in FIGs. 12 to 14 and the related descriptions thereof.

The imaging device 150 has a scanning cavity. The animal fixation device 110 and/or the animal chamber 130 are movable into the scanning cavity. The imaging device 150 is configured to scan the imaging animal(s) within the animal fixation device and/or the animal chamber 130. In some embodiments, the animal chamber 130 may be detachably mounted on a scanning bed of the imaging device 150. The animal chamber 130 is moved into the scanning cavity of the imaging device 150 by moving the scanning bed. In some embodiments, the animal chamber 130 may be integrally formed with the scanning bed of the imaging device 150. For example, the scanning bed may be directly configured in the form of a chamber body to serve as the animal chamber 130.

The imaging device 150 may include a single-modality imaging device, such as one of a Computed Tomography (CT) device, an MRI device, a Positron Emission Computed Tomography (PET) device, or a Single-Photon Emission Computed Tomography (SPECT) device. The imaging device 150 may include a multi-modality imaging device, such as a multi-modality imaging device formed by a combination of two or more of a CT device, an MRI device, a PET device, or a SPECT device (e.g., PET-CT device or PET-MRI device). In some embodiments, the imaging device 150 includes an emission computed tomography (ECT) device. The emission computed tomography in the present disclosure may include the PET, SPECT, multi-modality emission computed tomography (e.g., PET-CT), SPECT-CT, or the like.

The processing device 170 may obtain scan data from the imaging device 150 and process the scan data. For example, the processing device 170 may reconstruct a medical image for each imaging animal based on the scan data. As another example, when the imaging device 150 is a multi-modality scanning device, the processing device 170 may reconstruct images of different modalities and perform registration and fusion on these images. In some embodiments, the processing device 170 may be a single server or a server group.

In some embodiments, the processing device 170 may be integrated with the imaging device 150 as a single unit. In other embodiments, the processing device 170 may be independently arranged. For example, the processing device 170 may be local or remote. The processing device 170 may be implemented on a cloud platform (e.g., a private cloud, public cloud, hybrid cloud, or community cloud).

It should be noted that the animal imaging system 100 is provided for illustrative purposes only and is not intended to limit the scope of the present application. Those skilled in the art can make various modifications or variations based on the description of the present disclosure. For example, the animal imaging system 100 may further include a storage device for storing data and/or information obtained from other system components. However, such changes and modifications shall not depart from the scope of the present application.

FIG. 2 is a schematic diagram illustrating an exemplary animal tray according to some embodiments of the present disclosure.

As shown in FIG. 2, the animal tray 200 includes body part fixation portions 201 and marker accommodation portions 202. The body part fixation portions 201 are disposed at fixed positions on the animal tray and configured to fix body parts of an imaging animal placed on the animal tray, thereby fixing the positioning posture of the imaging animal. In some embodiments, the animal tray 200 includes the body part fixation portions 201, the marker accommodation portions 202, and a tray body 203. The tray body 203 is configured to place or accommodate the imaging animal. The body part fixation portions 201 are disposed at a fixed position on the tray body 203. In some embodiments, the tray body 203 and the body part fixation portions 201 of the tray 200 are integrally formed. For example, the tray body 203 and the body part fixation portions 201 are integrally formed by 3D printing or injection molding.

In some embodiments, the body part fixation portions 201 include a head fixation portion 210, a forelimb fixation portion 220, a hindlimb fixation portion 230, and a tail fixation portion 240. In some embodiments, an end of the animal tray 200 where the head fixation portion 210 is located is referred to as a first end portion (e.g., end E1 in FIG. 2), and an end where the tail fixation portion 240 is located is referred to as a second end portion (e.g., end E2 in FIG. 2).

The head fixation portion 210 is configured to fix the head of the imaging animal. In some embodiments, the head fixation portion 210 includes a head cap 211 and a bite bar 212. The head cap 211 is a cap-shaped structure for accommodating the head of the imaging animal. The bite bar 212 is for the teeth of the imaging animal (e.g., rodents such as mice or rabbits) to bite, thereby fixing the teeth of the imaging animal. The bite bar 212 has an opening (e.g., a circular opening or an opening of another shape) for hooking onto the teeth of the imaging animal. In some embodiments, the head fixation portion 210 includes ear bar(s) (not shown). An ear bar has a rod-shaped structure. By inserting the ear bar(s) into the ear canal(s) of the imaging animal and abutting against the skull of the imaging animal, fixation of the head of the imaging animal may be achieved. The head fixation portion 210 may include two ear bars, where one ear bar is for insertion into the left ear canal of the imaging animal, and the other ear bar is for insertion into the right ear canal of the imaging animal.

The forelimb fixation portion 220 is used for fixing the forelimbs of the imaging animal. In some embodiments, as shown in FIG. 2, the forelimb fixation portion 220 is designed as a groove structure. The groove structure provides sufficient operating space, facilitating an operator (e.g., laboratory personnel) to fix the forelimb of the imaging animal to the forelimb fixation portion 220 or to remove the forelimb from the forelimb fixation portion 220.

In some embodiments, the forelimb fixation portion 220 may be designed as a hole structure (as shown in FIG. 3). The hole structure has a cavity that penetrates through the forelimb fixation portion. After the forelimbs of the imaging animal are inserted into the cavity of the forelimb fixation portion, the forelimbs of the imaging animal may be effectively fixed under the constraint of the cavity. The hole structure is simple, easy to manufacture and process, not only making it easier to fix the forelimbs of the imaging animal but also avoiding compression on the forelimbs of the imaging animal. This makes subsequent imaging regions easier to distinguish and can lead to better imaging results.

The hindlimb fixation portion 230 is configured for fixing the hindlimbs of the imaging animal. Similar to the forelimb fixation portion 220, the hindlimb fixation portion 230 may be designed as a groove structure or a hole structure.

Animals used in animal experiments are typically quadrupeds. Accordingly, the animal tray 200 is provided with two forelimb fixation portions 220 and two hindlimb fixation portions 230 to fix the four limbs (i.e., two forelimbs and two hindlimbs) of the imaging animal.

The tail fixation portion 240 is configured to fix the tail of the imaging animal. It should be noted that for some imaging animals without tails (e.g., guinea pigs) or imaging animals with relatively short tails (where tail movement has a negligible effect on imaging, e.g., ferrets), the tail fixation portion 240 may be omitted. For some imaging animals with longer tails (e.g., mice), the tail fixation portion 240 is necessary.

In some embodiments, as shown in FIG. 2, the tail fixation portion 240 is designed as a groove structure. The groove structure provides sufficient operating space, facilitating an operator to fix the tail of the imaging animal to the tail fixation portion 240 or to remove the tail from the tail fixation portion 240. The tail fixation portion 240 can also be designed as other structures (not shown). For example, the tail fixation portion 240 may also be designed as a hole structure (i.e., a structure with an internal cavity). After the tail of the imaging animal is inserted into the cavity of the tail fixation portion 240, the tail of the imaging animal can be effectively fixed under the constraint of the cavity.

It should be noted that, in some scenarios, it is necessary to place an indwelling needle in the tail of the imaging animal. Due to the fixation effect of the tail fixation portion 240, the indwelling needle located in the tail of the imaging animal is also fixed, and the indwelling needle can be prevented from becoming ineffective due to displacement.

In some embodiments, the shape of the animal tray 200 corresponds to the shape of the imaging animal. In some embodiments, the dimensions of the surface of the animal tray 200 that contacts the imaging animal may be designed according to the body size of the imaging animal. Taking the imaging animal being a mouse as an example, based on rodent ergonomic design, when the mouse is placed in the animal tray 200, the body of the mouse can better conform to the animal tray 200, which helps achieve rapid fixation of various parts of the mouse, tail vein injection, positioning, and subsequent imaging.

In some embodiments, other auxiliary manners can also be employed to enhance the fixation of the imaging animal. For example, adhesive tape may be used to adhere the limbs and tail of the imaging animal to the corresponding fixation portions, to prevent the limbs and tail of the imaging animal from detaching from the corresponding fixation portions during the imaging process. As another example, adhesive tape may be used to adhere the body or a part of the body of the imaging animal to the tray, so that the body parts of the imaging animal are adequately fixed during the imaging process.

A marker accommodation portion 202 is configured to accommodate a marker (also referred to as a tracer). The marker is detectable in at least one imaging modality. The marker may be a solid or a liquid (e.g., water or positron nuclide-labeled glucose solution). The marker being detectable in at least one imaging modality refers to the marker being visible in medical images acquired by at least one imaging modality. The medical images include one or more of MR images, PET images, CT images, and SPECT images. In some embodiments, the marker is detectable in at least one first imaging modality. The at least one first imaging modality includes at least one of positron emission imaging, single-photon emission imaging, or MRI. In some embodiments, the marker accommodation portion 202 is detectable in at least one second imaging modality. The at least one second imaging modality includes X-ray imaging. For example, the marker accommodation portion 202 may be made of plastic. The X-ray imaging may include Digital Radiography (DR) and CT imaging.

In some embodiments, the marker accommodation portions 202 include a first marker accommodation portion 250 and a second marker accommodation portion 260. The first marker accommodation portion 250 is disposed at a first position on the animal tray 200 proximate to the forelimb fixation portion 220. The second marker accommodation portion 260 is disposed at a second position on the animal tray 200 proximate to the hindlimb fixation portion 230. In some embodiments, the first marker accommodation portion 250 is disposed at the first position on the tray body 203 proximate to the forelimb fixation portion 220. The second marker accommodation portion 260 is disposed at the second position on the tray body 203 proximate to the hindlimb fixation portion 230. The tray body 203 of the tray 200 may be integrally formed with the body part fixation portions 201 and the marker accommodation portions 202, or the tray body 203, the body part fixation portions 201, and the marker accommodation portions 202 may be detachable and configurable for assembly. In some embodiments, the tray body 203 and the marker accommodation portion 202 are separate parts that can be assembled or disassembled.

Since the marker is detectable in medical images, with the aid of the first marker accommodation portion 250 and the second marker accommodation portion 260, by setting a plurality of markers, the fusion accuracy of multi-modality images can be improved, achieving rapid registration and addressing pain points in the prior art such as image fusion accuracy being limited by mechanical structure motion precision and imaging animal positioning. Furthermore, since the first marker accommodation portion 250 and the second marker accommodation portion 260 are disposed at the four corners of the animal tray 200, this design not only maximizes the spatial utilization of the animal tray 200 but also prevents any interference with the body of the imaging animal. Consequently, the marker stands out more prominently in medical images, which facilitates localization.

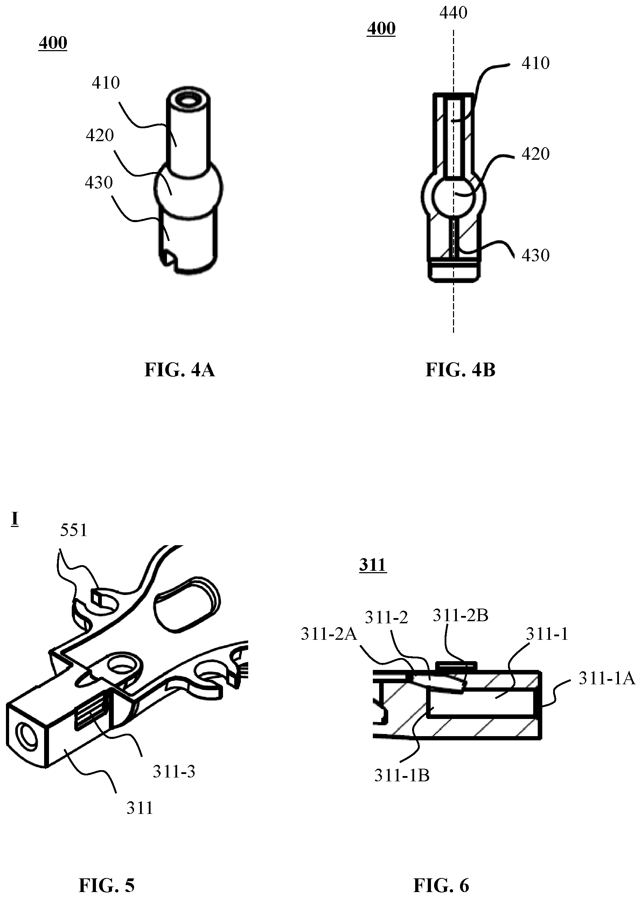

In some embodiments, the first marker accommodation portion 250 and the second marker accommodation portion 260 may be fixedly installed on the animal tray 200, or may be detachably installed on the animal tray 200. In some embodiments, the structure of the first marker accommodation portion 250 and/or the second marker accommodation portion 260 is the same as or similar to the structure of the marker accommodation portion 400 shown in FIGs. 4A and 4B, and the marker therein may be replaceable.

In some embodiments, the marker accommodation portions 202 include a third marker accommodation portion 270, which is disposed at a third position near the center of the animal tray 200. As shown in FIG. 2, one third marker accommodation portion 270 is respectively disposed on each side near the center position of the animal tray 200. In some embodiments, the third marker accommodation portion 270 may be detachably connected to the animal tray 200. In some embodiments, the third marker accommodation portion 270 may be fixedly installed on the animal tray 200 or integrally formed with the animal tray 200.

In some embodiments, the structure of the third marker accommodation portion 270 is similar to that of the marker accommodation portion 400 shown in FIGs. 4A and 4B. In some embodiments, the third marker accommodation portion 270 may be configured as an internally hollow sphere, cylinder, or other regular or irregular sealed cube. The marker accommodated therein may be pre-placed. For example, a solid or liquid (such as water) that is detectable in MR images and does not decay may be pre-placed in the third marker accommodation portion 270. In some embodiments of the present disclosure, by integrally forming the third marker accommodation portion with the animal tray or fixedly connecting the third marker accommodation portion to the animal tray, the overall structural complexity of the animal tray may be reduced, lowering costs. Pre-placing the marker can eliminate the work of replacing the marker, improving scanning efficiency.

It should be noted that the arrangement of the marker accommodation portion(s) may be determined according to actual needs. For example, when the animal tray is used for a plurality of MR scans on a plurality of body segments of the imaging animal, the arrangement of the marker accommodation portion(s) may be determined based on the imaging region of the MR scan, the region covered by each segment of the preset multi-segment scans performed on the imaging animal, etc. For example, when only two-segment scans on the upper body and lower body of the imaging animal are needed, the first marker accommodation portion 250 and the second marker accommodation portion 260 in FIG. 2 may be omitted. As another example, a plurality of third marker accommodation portions 270 may be arranged on both sides of the animal tray 200, and the third marker accommodation portions 270 on each side may be arranged at preset intervals. For example, in different scans, different parts of the animal tray 200 may be located in the imaging region of the MRI device, so the third marker accommodation portion(s) within the imaging region may also differ. By setting the plurality of third marker accommodation portions, it can be ensured that at least one third marker accommodation portion is located within the imaging region in each scan, providing more reference for subsequent image registration/stitching and improving the accuracy of registration/stitching.

FIG. 3 is a schematic diagram illustrating another exemplary animal tray according to some embodiments of the present disclosure. The animal tray 300 is similar to the animal tray 200, except for certain components or features.

As shown in FIG. 3, the first end portion E1 of the animal tray 300 is provided with a bite bar 212, the second end portion E2 is provided with a tail fixation portion 240, and both sides are provided with third marker accommodation portions 270. The animal tray 300 is provided with a first hole structure 330 and a second hole structure 340 that penetrate through the animal tray 300. The first hole structure 330 serves as a forelimb fixation portion. The second hole structure 340 serves as a hindlimb fixation portion. In some embodiments, the first hole structure 330 and the second hole structure 340 may be waist-shaped holes. The waist-shaped holes have a simple structure and can accommodate various imaging animals whose limb dimensions fall within a certain size range.

In some embodiments of the present disclosure, by fixing the forelimbs and hindlimbs of the imaging animal through hole structures, it is easier to achieve fixation of the animal's forelimbs and hindlimbs, which is beneficial for the animal to present a preset positioning (e.g., orientation) or posture. In addition, it avoids compressing the forelimbs and hindlimbs of the imaging animal, and reduces contact between different parts (e.g., due to movement of the forelimb(s) and/or hindlimb(s) overlapping with body parts). Furthermore, this arrangement avoids image artifacts caused by the movement of the imaging animal's limbs (e.g., the animal's forelimbs and hindlimbs) affecting body parts during imaging, and also facilitates separate imaging and structural analysis of the limbs or body parts of the imaging animal.

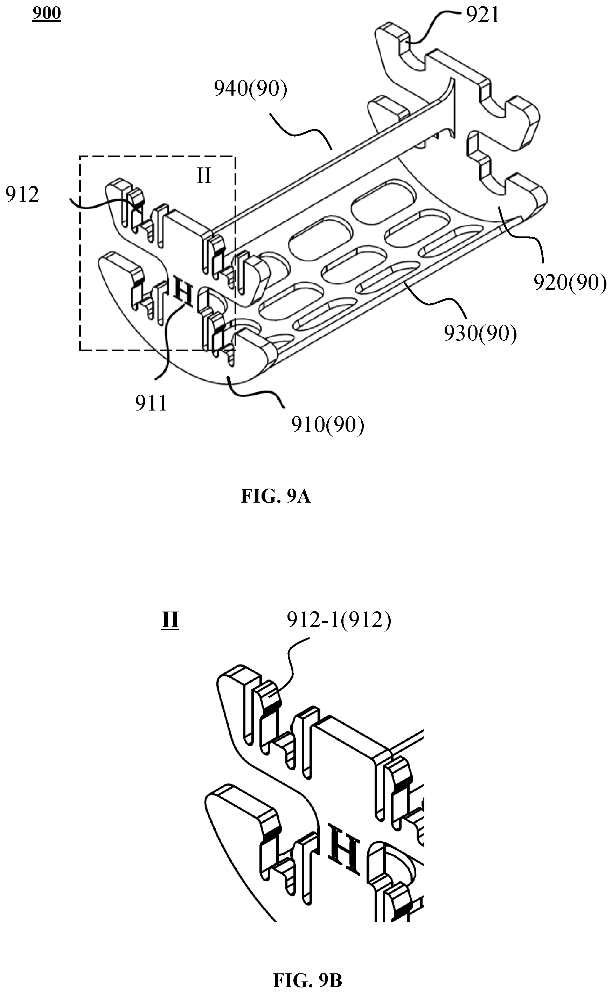

In some embodiments, the second end portion E2 further includes a card slot 280 configured to engage with a card slot structure of a support portion. More descriptions regarding the support portion may be found in FIG. 9A and related descriptions thereof.

The first end portion E1 of the animal tray 300 for placing the head of the imaging animal is further provided with an anesthesia interface 311 configured to deliver anesthetic gas to the imaging animal for anesthetizing the imaging animal. More descriptions regarding the anesthesia interface 311 may be found in FIGs. 5 and 6 and related descriptions thereof.

The animal tray 300 is further provided with fixing structures 350. Each marker accommodation portion is detachably mounted to the animal tray 300 via one fixing structure 350. A marker accommodation portion may be connected to a fixing structure 350 by means of a threaded connection, a snap-fit connection, or the like. There may be a plurality of fixing structures 350. For example, as shown in FIG. 3, the animal tray 300 may be provided with two fixing structures 350 at positions near the first end portion E1 and two fixing structures 350 at positions near the second end portion E2.

In some embodiments, a fixing structure 350 is a clamping ring structure. The clamping ring structure includes elastic clamping ring arms configured to secure a marker accommodation portion. Referring to the enlarged schematic diagram of Region I in FIG. 5, the clamping ring structure includes elastic clamping ring arms 551. The clamping ring arms 551 are configured to secure the marker accommodation portion. The structure of the marker accommodation portion matches the clamping ring structure. During installation, the marker accommodation portion may be inserted into the clamping ring structure through the opening of the clamping ring arms 551 and be clamped by the clamping ring arms 551, thereby achieving fixation of the marker accommodation portion. The fixation method using the clamping ring structure facilitates the detachment and installation of the marker accommodation portion. The marker accommodation portion may be configured according to experimental needs, set for one-time use or repeated use, while the main structure of the animal tray itself can be used continuously.

FIGs. 4A and 4B are schematic diagrams illustrating an exemplary marker accommodation portion according to some embodiments of the present disclosure.

As shown in FIGs. 4A and 4B, the marker accommodation portion 400 includes a first accommodation channel 410, a second accommodation channel 430, and a marker accommodation cavity 420. The first accommodation channel 410 is configured for injecting the marker. The second accommodation channel 430 is configured for expelling gas during injection of the marker. The marker accommodation cavity 420 is respectively in communication with the first accommodation channel 410 and the second accommodation channel 430 and is configured to accommodate the marker.

In some embodiments, the first accommodation channel 410 and the second accommodation channel 430 are cylindrical, and their interiors are hollow. In some embodiments, the first accommodation channel 410 and the second accommodation channel 430 may have other shapes, e.g., internally hollow cuboids, triangular prisms, or the like. In some embodiments, the shapes of the first accommodation channel 410 and the second accommodation channel 430 may be the same or different. In some embodiments, the marker accommodation cavity 420 may be spherical, and its interior is hollow. For example, a spherical shape with a diameter of 4 mm. In some embodiments, the marker accommodation cavity 420 may have other shapes, e.g., internally hollow cuboids, cubes, or the like.

In some embodiments, a center of the marker accommodation cavity 420 is located on a central axis of the first accommodation channel 410 and/or the second accommodation channel 430. As shown in FIG. 4B, the central axis of the first accommodation channel 410 and the second accommodation channel 430 is an axis 440. The center of the marker accommodation cavity 420 is located on the axis 440. By arranging the center of the marker accommodation cavity 420 to be located on the central axis of the first accommodation channel 410, registration during multi-modality imaging may be facilitated, improving registration efficiency and accuracy. In some embodiments of the present disclosure, by coaxially arranging the first accommodation channel, the second accommodation channel, and the marker accommodation cavity, the marker and the marker accommodation portion that are detectable in medical images of different modalities have the same central axis, thereby enabling precise fusion of multi-modality images with the aid of the marker and the marker accommodation portion.

In some embodiments, the marker accommodation portion 400 is formed by 3D printing. In some embodiments, the marker accommodation portion 400 can be formed by other methods, e.g., injection molding.

In some embodiments, a length of the first accommodation channel 410 is greater than a length of the second accommodation channel 430, and a diameter of the first accommodation channel 410 is greater than a diameter of the second accommodation channel 430. The diameter of an accommodation channel refers to the diameter of its hollow part. For example, the length of the first accommodation channel 410 may be 7.5 mm, and the diameter of the first accommodation channel 410 may be 1.6 mm; the diameter of the second accommodation channel 430 may be 0.6 mm.

In some embodiments of the present disclosure, by setting the length of the first accommodation channel to be greater than that of the second accommodation channel, the lengthened first accommodation channel can help laboratory personnel control the injection of the marker more precisely, preventing the marker from overflowing during the injection process. Furthermore, by setting the diameter of the second accommodation channel to be smaller than that of the first accommodation channel, the reduced diameter of the second accommodation channel allows gas to be expelled smoothly during marker injection, and due to surface tension at the outlet of the second accommodation channel, the marker solution can be prevented from flowing out of the second accommodation channel.

FIG. 6 is a schematic diagram illustrating an exemplary anesthesia interface according to some embodiments of the present disclosure.

As shown in FIG. 6, the anesthesia interface 311 includes a first anesthesia channel 311-1 and a second anesthesia channel 311-2. The first anesthesia channel 311-1 includes a first end 311-1A and a second end 311-1B. The second anesthesia channel 311-2 includes a third end 311-2A and a fourth end 311-2B. The first end 311-1A is an inlet for anesthetic gas. The third end 311-2A is an outlet for the anesthetic gas and is located near a placement position of the nose of the imaging animal. By setting the third end at the placement position of the nose of the imaging animal, the anesthetic gas can be absorbed by the imaging animal at the outlet position, which is conducive to improving the anesthetic effect.

In some embodiments, the second end 311-1B is in communication with the fourth end 311-2B. At the communication point, a preset angle is formed between the second end 311-1B and the fourth end 311-2B. For example, the preset angle may be 30°. In some embodiments, the preset angle may be other angles, e.g., 25° or 35°. Merely by way of example, as shown in FIG. 6, anesthetic gas may be injected into the anesthesia interface 311 through the first end 311-1A, pass through the first anesthesia channel 311-1 and the second anesthesia channel 311-2, flow out from the third end 311-2A, and anesthetize the imaging animal.

In some embodiments of the present disclosure, by providing the anesthesia interface and setting the preset angle between the second end and the fourth end, the anesthetic gas can be directed directly towards the nose of the imaging animal after passing through the first anesthesia channel and the second anesthesia channel, without the need for additional pipes or accessories. This makes the anesthesia apparatus more concise and can also improve the anesthetic effect.

FIG. 7 is a schematic diagram illustrating another exemplary animal tray according to some embodiments of the present disclosure. The animal tray 700 is similar to the animal trays 200 and 300, except for certain components or features. As shown in FIG. 7, the first end portion E1 of the animal tray 700 is provided with a bite bar 212, the second end portion E2 is provided with a tail fixation portion 240, and the main body portion is provided with first hole structures 330, second hole structures 340, and fixing structures 350.

The animal tray 700 further includes a physiological signal acquisition device configured to acquire a physiological signal of the imaging animal. The physiological signal includes, but is not limited to, a heartbeat signal, a pulse signal, or the like.

In some embodiments, the physiological signal acquisition device includes a respiration signal acquisition device 710 as shown in FIG. 7. The respiration signal acquisition device 710 includes a gas tube (not shown in the figure), one end of the gas tube is disposed at the first end portion E1 of the animal tray 700 for placing the head of the imaging animal and is configured to guide out exhaled gas of the imaging animal; an airbag 711, disposed at a bottom of the animal tray 700 and in communication with the other end of the gas tube; and a pressure sensor (not shown in the figure) configured to detect a pressure change within the airbag 711 and convert the pressure change into a respiration signal. The respiration signal is used to reflect a respiration state (e.g., exhalation, inhalation) of the imaging animal.

In some embodiments, the gas tube and/or the airbag may be omitted. For example, the physiological signal acquisition device includes a pressure sensor, wherein the pressure sensor is in fixed contact (e.g., bound) with a side of the chest/abdomen of the imaging animal. The heartbeat state and/or respiration state of the imaging animal is determined based on changes in the pressure signal collected by the pressure sensor.

In some embodiments of the present disclosure, the respiration signal acquisition device can monitor the respiration state of the imaging animal during the scanning process, thereby enabling subsequent gated reconstruction of the scan data to reduce artifacts caused by respiratory motion. Furthermore, gated scanning can be performed based on the respiration condition of the imaging animal, thereby reducing the radiation dose to the imaging animal and/or the operator.

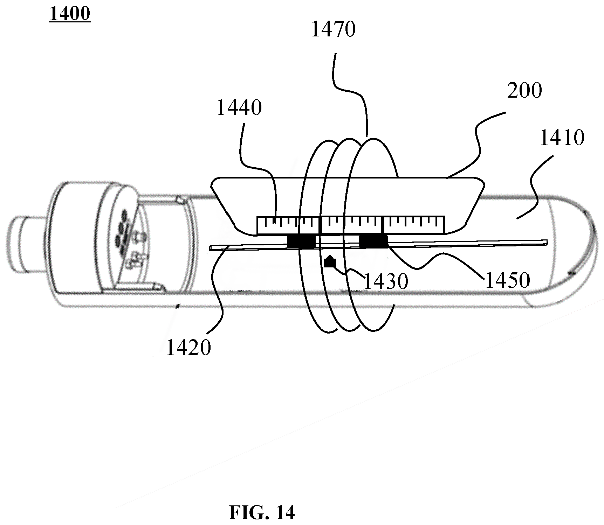

The animal tray 700 further includes a receiving coil 720. The receiving coil 720 is embedded in a bottom of the animal tray 700 and is configured to acquire MR data during an MR scan of the imaging animal. In some embodiments, the bottom of the animal tray 700 is provided with a mounting channel to allow the receiving coil 720 to be embedded within the animal tray 700. The receiving coil 720 may be configured as a single-loop coil or an array coil. In some embodiments, the receiving coil 720 is disposed around the airbag 711 of the respiration signal acquisition device 710, thereby better acquiring MR data of the abdomen or heart of the imaging animal. In some embodiments, a plurality of animal trays 700 equipped with receiving coils 720 may be placed on a multi-imaging-animal fixation device. Each receiving coil 720 may separately acquire MR data from the corresponding imaging animal. In the prior art, receiving coils are typically installed on the exterior of an animal chamber (similar to what is shown in FIG. 14), making simultaneous MRI of the plurality of imaging animals impossible. The animal tray 700 in the present disclosure can achieve simultaneous MRI of the plurality of imaging animals, improving imaging efficiency.

In some embodiments of the present disclosure, by embedding the receiving coil in the bottom of the animal tray, the work of installing the receiving coil can be eliminated, improving scanning efficiency. Furthermore, combining the physiological signal acquisition device with the receiving coil can enable respiratory gating during the MR scanning process, which helps eliminate the impact of respiratory motion of the imaging animal on MR image quality (e.g., reducing respiratory artifacts).

It should be understood that the animal trays in FIGs. 2, 3, and 7 and the related descriptions are for illustrative purposes only and are not intended to be limiting. Features in the animal trays 200, 300, 700 may be combined in any manner according to practical needs. For example, the animal tray 200 may include the fixing structures 350 as shown in FIG. 3, and the forelimb fixation portion and the hindlimb fixation portion in the animal tray 300 may be configured as the groove structures as shown in FIG. 2. As another example, the animal trays 200 and 300 may further include the respiration signal acquisition device 710 and the receiving coil 720 as shown in FIG. 7.

FIG. 8 is a schematic diagram illustrating an exemplary multi-imaging-animal fixation device according to some embodiments of the present disclosure.

As shown in FIG. 8, the multi-imaging-animal fixation device 800 includes a plurality of animal trays 200 and a support portion 801. The support portion 801 is configured to support the plurality of animal trays 200. Each animal tray 200 includes a first end portion E1 and a second end portion E2. The first end portion E1 is configured to place the head of an imaging animal, and the second end portion E2 is disposed opposite to the first end portion E1. The support portion 801 includes a first portion 810 and a second portion 820 disposed opposite to each other. The first portion 810 is configured to support the first end portions E1 of the plurality of animal trays 200. The second portion 820 is configured to support the second end portions E2 of the plurality of animal trays 200. The support portion 801 further includes a third portion 830 configured to connect the first portion 810 and the second portion 820.

In some embodiments, the plurality of animal trays in the multi-imaging-animal fixation device are arranged in at least two layers, and the animal trays in different layers are connected via the support portion. As shown in FIG. 8, the multi-imaging-animal fixation device 800 includes four animal trays 200 connected by the support portion 801. The four animal trays 200 are arranged in two layers, with two animal trays 200 in each layer. In some embodiments, the number of animal trays 200 connected to the support portion 801 may be in the range of 2 to 4. In some embodiments, the support portion 801 and the animal trays 200 are detachably connected via, e.g., a snap-fit connection, a card slot connection, or the like.

In some embodiments, a first recess is formed in the bottom of the head fixation portion 210 of the animal tray 200, and an edge of the first portion 810 is inserted into the first recess to achieve support for the animal tray 200. In some embodiments, a second recess is formed in a bottom of the tail fixation portion 240 of the animal tray 200, and an edge of the second portion 820 is inserted into the second recess to achieve support for the animal tray 200. In some embodiments, a first recess is formed at an edge of the first portion 810, and the head fixation portion 210 is inserted into the first recess to receive support from the first portion 810. Similarly, a second recess is formed at an edge of the second portion 820, and the tail fixation portion 240 is inserted into the second recess to receive support from the second portion 820.

By combining support portions of different structures in different ways, different usage requirements may be met. For example, referring to FIG. 8, both the first portion 810 and the second portion 820 retain only the lower halves (e.g., omitting the upper T-shaped structures) to achieve a combination of two animal trays 200 in a single layer. As another example, referring to FIG. 8, when the second layer is changed to have only one animal tray, the upper ends of the first portion 810 and the second portion 820 may be designed shorter (e.g., simplified from a T-shaped structure to a handle-like structure).

In some embodiments, referring to FIG. 8, the support portion further includes the third portion 830 between the first portion 810 and the second portion 820. With the third portion 830, the plurality of animal trays 200 can be more stably combined, and overall movement of the multi-imaging-animal fixation device 800 is facilitated.

It should be understood that the multi-imaging-animal fixation device 800 may also be used to support other animal trays disclosed in the present disclosure, e.g., the animal tray 300 shown in FIG. 3.

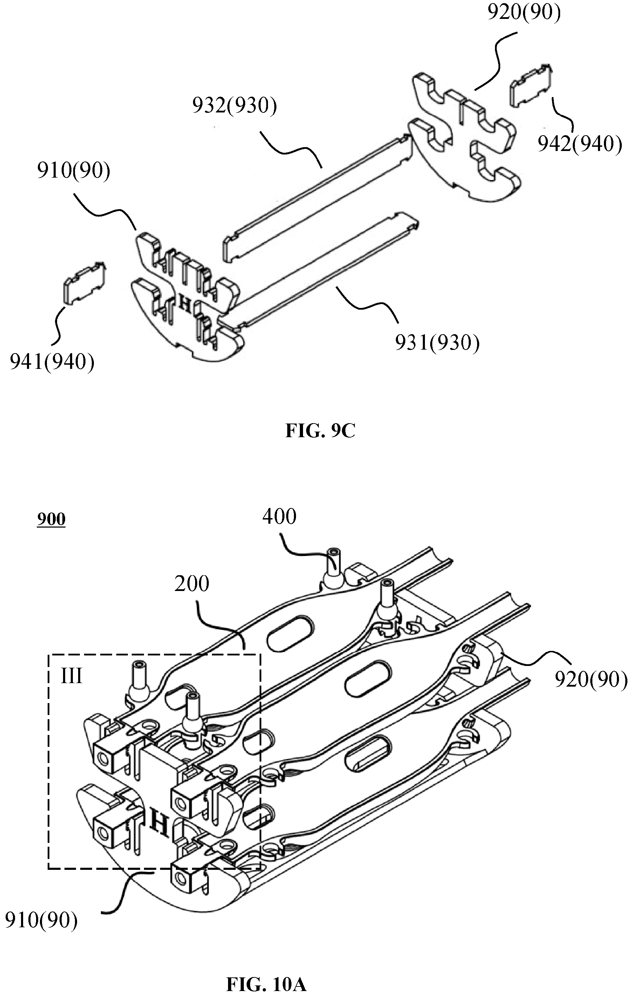

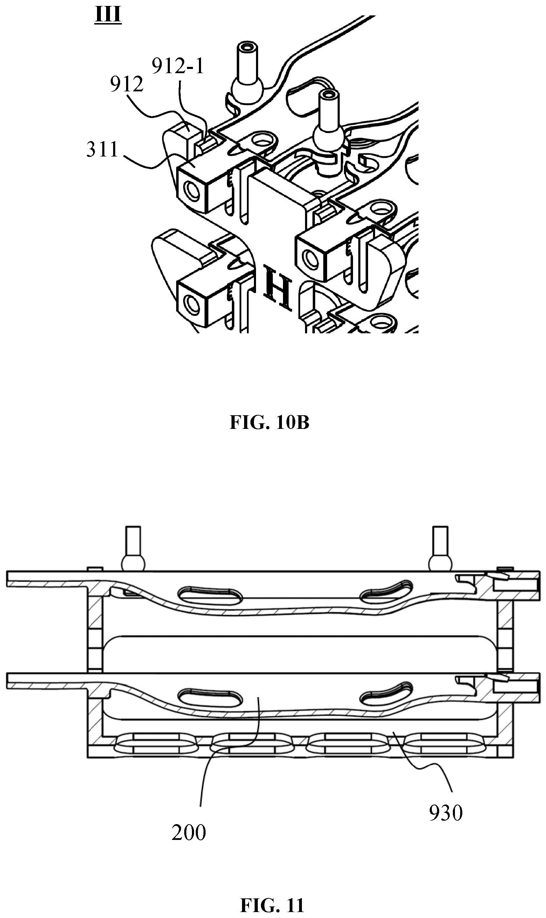

FIG. 9A is a schematic diagram illustrating an exemplary support portion of a multi-imaging-animal fixation device according to some embodiments of the present disclosure. FIG. 9B is an enlarged schematic diagram illustrating Region II of the support portion. FIG. 9C is a schematic diagram illustrating a disassembled support portion according to some embodiments of the present disclosure. FIG. 10A is a schematic diagram illustrating a multi-imaging-animal fixation device after animal trays are installed on the support portion according to some embodiments of the present disclosure. FIG. 10B is an enlarged schematic diagram illustrating Region III in FIG. 10A according to some embodiments of the present disclosure. FIG. 11 is a cross-sectional schematic diagram illustrating an exemplary multi-imaging-animal fixation device according to some embodiments of the present disclosure.

As shown in FIGs. 9A and 10A, the multi-imaging-animal fixation device 900 includes a support portion 90, and the support portion 90 is configured to support a plurality of animal trays 200. The support portion 90 includes a first portion 910 and a second portion 920 disposed opposite to each other. The first portion 910 is configured to support the first end portions of the animal trays 200. The second portion 920 is configured to support the second end portions of the animal trays 200.

In some embodiments, the first portion 910 is provided with an indication mark 911 for indicating placement positions for the first end portions of the plurality of animal trays, which facilitates a user to quickly locate the positions for installing the first end portions of the animal trays. The indication mark 911 may be represented in various ways, e.g., by color, by characters, by patterns, etc. Merely by way of example, as shown in FIG. 9A, the indication mark 911 is the character "H".

In some embodiments, as shown in FIGs. 9A and 10A, the first portion 910 is provided with engagement structures 912, and the engagement structures 912 are configured to engage with the first end portions E1 of the animal trays 200. In some embodiments, the second portion 920 is provided with card slot structures 921, and the card slot structures 921 are configured to engage with the second end portions E2 of the animal trays 200. For example, the card slot structures 921 of the second portion 920 engage with card slots (e.g., card slot 280) of the animal trays. The card slot structures 921 and the card slots may have various shapes, provided that their shapes match to achieve engagement.

In some embodiments, referring to FIG. 9B and FIG. 5, an engagement structure 912 includes an outwardly protruding snap-fit boss 912-1. The anesthesia interface 311 is provided with an inwardly recessed snap-fit structure 311-3. The snap-fit boss 912-1 is configured to be embedded into the snap-fit structure 311-3 to achieve snap-fit engagement between the anesthesia interface 311 and the engagement structure 912. The engagement structure 912 has a certain elasticity. The anesthesia interface 311 may be inserted into the engagement structure 912 through an opening of the engagement structure 912. Installation is completed when the snap-fit boss 912-1 is embedded in the snap-fit structure 311-3.

As shown in FIG. 10B, after the snap-fit boss 912-1 is embedded into the snap-fit structure 311-3, the snap-fit boss 912-1 abuts against the anesthesia interface 311, and its protruding portion can restrict movement of the anesthesia interface 311, thereby achieving the purpose of fixing the anesthesia interface 311 and further realizing stable installation of the animal tray. The snap-fit boss 912-1 and the snap-fit structure 311-3 may have various shapes, provided that their shapes match to achieve engagement.

In some embodiments of the present disclosure, providing a snap-fit boss on the engagement structure and a snap-fit structure on the anesthesia interface enables quick and convenient installation of the animal tray onto the support portion and enhances installation stability.

In some embodiments, the support portion 90 further includes a third portion 930 located between the first portion 910 and the second portion 920. The third portion 930 is provided with hollowed-out portions. The hollowed-out portions may include various shapes, e.g., squares or circles. Merely by way of example, as shown in FIG. 9A, the third portion 930 is provided with a plurality of hollowed-out portions. Providing hollowed-out portions on the third portion of the support portion helps reduce the weight of the support portion, thereby reducing the weight of the multi-imaging-animal fixation device. In some embodiments, as shown in FIG. 11, the bottom of the lowermost animal tray 200 has a certain spacing from the third portion 930 of the support portion to ensure that the limbs of the imaging animal are not compressed and can extend naturally.

In some embodiments, as shown in FIG. 9C, the third portion 930 includes a support base 931 and a support beam 932. The support beam 932 is suspended between and connected to the first portion 910 and the second portion 920. The support base 931 is disposed at a bottom of the multi-imaging-animal fixation device 900 and is configured to support the first portion 910, the second portion 920, and the support beam 932.

In some embodiments, the first portion 910, the second portion 920, and the third portion 930 are independent components capable of being assembled to form the support portion. The first portion 910, the second portion 920, and the third portion 930 may be combined into one unit through a manner similar to block building.

In some embodiments, the support portion 90 further includes connectors 940 disposed on the first portion and/or the second portion (e.g., a connector 941 and/or a connector 942). The connectors 940 are configured to connect the multi-imaging-animal fixation device to another imaging animal fixation device. Through the connectors on the support portion, a plurality of different imaging animal fixation devices may be combined and connected in the vertical direction and/or the front-rear axis direction, thereby satisfying experimental needs for more imaging animals. Merely by way of example, a first connector and a second connector are respectively provided on the multi-imaging-animal fixation device and the other imaging animal fixation device. The first connector and the second connector are structurally complementary to achieve a connection, such as a snap-fit connection, threaded connection, plug connection, etc. Through the first connector and the second connector, two multi-imaging-animal fixation devices may be spliced together.

FIG. 12 is a schematic diagram illustrating an exemplary animal chamber according to some embodiments of the present disclosure. As shown in FIG. 12, the animal chamber 1200 includes a chamber body 1210 and the multi-imaging-animal fixation device 800. FIG. 13 is a schematic diagram illustrating another exemplary animal chamber according to some embodiments of the present disclosure. As shown in FIG. 13, the animal chamber 1300 includes a chamber body 1310 and the multi-imaging-animal fixation device 900. The multi-imaging-animal fixation device 900 and the multi-imaging-animal fixation device 800 shown in the figures can each simultaneously accommodate 4 animal trays. In some embodiments, for larger imaging animals (e.g., guinea pigs), a multi-imaging-animal fixation device capable of accommodating 2 animal trays may be provided, with the 2 animal tray positions arranged vertically.

In some embodiments, the third portion of the support portion has an arcuate bottom surface, and the arcuate bottom surface matches an inner wall of the chamber body. In some embodiments, a radius of curvature of the arcuate bottom surface of the support portion is the same as a radius of curvature of the inner wall of the chamber body. The multi-imaging-animal fixation device, including the support portion and the animal trays can be directly placed within the chamber body without additional fixing structures to secure the multi-imaging-animal fixation device. This arrangement further improves assembly efficiency and has better applicability.

A traditional animal chamber has slots formed in the inner wall for fixing imaging animals. On one hand, the slots cannot separate from the animal chamber to fix the imaging animals; the animal chamber cannot be used for experimental preparation (e.g., fixing the animals to be scanned) of the animals to be scanned while in use, and due to the large volume of the animal chamber, which occupies more space in scanning scenarios. On the other hand, the slot design of a single animal chamber is fixed, which is not conducive to flexibly supporting various simultaneous scanning modes (especially multi-layer simultaneous scanning modes).

The animal trays and multi-imaging-animal fixation devices provided by the embodiments of the present disclosure separate the centralized storage function and the auxiliary imaging function of traditional animal chambers, making the described arrangements more space-saving in scanning scenarios and supporting simultaneous scanning modes (especially multi-layer simultaneous scanning modes). Furthermore, by leveraging flexible and versatile multi-imaging-animal fixation devices (especially those with multi-layer structures), the animal chambers provided by the embodiments of the present disclosure can centrally store more fixed imaging animals. Moreover, the single/multi-imaging-animal fixation devices of the present disclosure themselves possess fixation and positioning functions, feature simple structures, are easy to manufacture, and can be easily placed within the chamber bodies of existing animal chambers. The devices are not only applicable to various types of animal chamber bodies and scanning equipment but also allow a single animal imaging system to be configured with a plurality of multi-imaging-animal fixation devices and/or various models of multi-imaging-animal fixation devices to satisfy different user needs.

In some embodiments, an animal chamber includes one or more multi-imaging-animal fixation devices. Each of the one or more multi-imaging-animal fixation devices may be configured with a plurality of animal trays. The one or more multi-imaging-animal fixation devices may be all identical, partially identical, or all different models. The plurality of animal trays may be all identical, partially identical, or all different models. When the one or more multi-imaging-animal fixation devices are placed in an animal chamber, they may be arranged along the axial (i.e., longitudinal) direction of the animal chamber, thereby further increasing the number of imaging animals that can be scanned simultaneously. For example, with one multi-imaging-animal fixation device having 4 animal tray positions, placing two multi-imaging-animal fixation devices in one animal chamber enables simultaneous scanning of 8 imaging animals.

FIG. 14 is a schematic diagram illustrating another exemplary animal chamber according to some embodiments of the present disclosure. The animal chamber shown in FIG. 14 may be configured for MRI. As shown in FIG. 14, the animal chamber 1400 includes a chamber body 1410. The chamber body 1410 may accommodate the animal tray 200. An MR coil 1470 is installed on an exterior of the chamber body 1410. The MR coil 1470 refers to a receiving coil used for MRI, which may be various types of coils, such as a volume coil, an array coil, or a surface coil. For example, the MR coil 1470 may be a 42 mm volume coil, which may be mounted around the exterior of the animal chamber 1400.

In some embodiments, as shown in FIG. 14, the animal chamber 1400 is provided with an indication mark 1430 for indicating a center of the MR coil 1470. The indication mark 1430 assists in placement and movement of the animal tray 200 within the animal chamber 1400 to align an imaging site of the imaging animal with the center of the MR coil 1470 before scanning. For example, when scanning a first part of the imaging animal, the first part needs to be aligned with the indication mark 1430, thereby aligning the first part with the center of the MR coil 1470. When scanning a second part of the imaging animal, the second part needs to be aligned with the indication mark 1430, thereby aligning the second part with the center of the MR coil 1470.

The indication mark 1430 may be any visual mark. For example, the indication mark 1430 may be a symbol (e.g., a colored arrow or a dot) or a marker engraved at a preset position on the animal chamber 1400. In some embodiments, the indication mark 1430 may be set at a preset position (e.g., a central position along the longitudinal axis or another position) on the exterior of the animal chamber 1400. In some embodiments, the indication mark 1430 may be set at a coil mounting structure. The coil mounting structure (not shown in the figure) is used to mount and secure the MR coil 1470, so that the MRI device is able to scan the imaging site of the imaging animal within the animal chamber 1400. The coil mounting structure may be pre-configured based on information (e.g., a type and a size) of the MR coil 1470 used in the experiment, so that the coil mounting structure can connect and/or install the MR coil 1470. In some embodiments, the coil mounting structure may be disposed at one or more different positions on the animal chamber 1400 based on scanning requirements for the imaging animal (e.g., single-segment or multi-segment scans, a coverage area for whole-body or partial scanning of the animal).

In some embodiments, continuing to refer to FIG. 14, the animal tray 200 is provided with a scale indicator device 1440. The scale indicator device 1440 and the indication mark 1430 can cooperate to assist in placement and movement of the animal tray 200 within the animal chamber 1400. In some embodiments, the scale indicator device 1440 may be disposed on two side surfaces of the animal tray 200.

The scale indicator device 1440 may be in the form of a scale, which includes scale readings. Different scale readings correspond to different parts of the imaging animal. For example, the scale increases progressively starting from the first end portion of the animal tray 200, meaning a smaller scale reading indicates a location closer to the head of the imaging animal. When the imaging animal is placed on the animal tray 200, the relative positions of the imaging site of the imaging animal and/or a plurality of points within the imaging site on the animal tray 200 may be determined based on the scale indicator device 1440. Furthermore, when the animal tray 200 moves within the animal chamber 1400, the scale reading on the scale indicator device 1440 pointed to by the indication mark 1430 (hereinafter referred to as the current scale reading) may be used to determine the relative positional relationship of the imaging site and/or the plurality of points within the imaging site with respect to the center point of the MR coil 1470. Merely by way of example, the scale indicator device 1440 includes scale readings from 0 to 10. The scale reading 5 being aligned with the indication mark 1430 denotes that a center point of the imaging animal is aligned with the center point of the MR coil 1470. As another example, the scale reading 2.5 being aligned with the indication mark denotes that the center point of the upper body (e.g., the chest center point) of the imaging animal is aligned with the center point of the MR coil 1470.

It should be noted that a length of the animal chamber 1400 is greater than a length of the animal tray 200. For example, the length of the animal chamber 1400 is a preset multiple (e.g., 1.5 times or 2 times) of the length of the animal tray 200, so that the animal tray 200 has sufficient space to be placed therein and can move along its longitudinal axis within a preset distance range (e.g., 0.5 times or 1 times the length of the animal tray).

In some embodiments, an operator can determine whether the imaging region is located within a scanning region or imaging region of the MRI device through the indication mark 1430 in the animal chamber 1400 and the scale indicator device 1440 on the animal tray 200, thereby assisting in determining the moving direction and/or moving distance of the animal tray 200 within the animal chamber. Merely by way of example, for scanning a first part (e.g., the upper body) of the imaging animal, the operator can move the animal tray 200 so that the center of the MR coil 1470 aligns with the center position of the first part of the imaging animal. For example, the animal tray 200 may be moved so that the scale reading 2.5 aligns with the indication mark 1430, thereby allowing subsequent scanning operations by the MRI device to be performed.

In some embodiments of the present disclosure, the scale indicator device on the animal tray and the indication mark on the animal chamber enable more precise movement of the animal tray by the operator, making subsequent MR scans of different parts of the imaging animal more targeted.

In some embodiments, as shown in FIG. 14, the animal chamber 1400 is provided with a slide rail 1420 extending along a longitudinal axis of the animal chamber 1400. The animal tray 200 is provided with a slider 1450 capable of sliding along the slide rail 1420. The animal tray 200 and the animal chamber 1400 are slidably connected via the slide rail 1420 and the slider 1450, so that the animal tray 200 is movable within the animal chamber 1400 along the longitudinal axis of the animal chamber 1400.

The slide rail 1420 may be configured to guide sliding or movement of the animal tray 200 within the animal chamber 1400. The slide rail 1420 may be in the form of various guiding devices, such as a guide rail, a track, or a guide groove. Merely by way of example, as shown in FIG. 14, the slide rail 1420 may be disposed at a bottom of the chamber body 1410 and extend along the longitudinal axis of the animal chamber 1400. A longitudinal center line of the slide rail 1420 may coincide with a longitudinal center line of the animal chamber 1400.

The slider 1450 on the animal tray 200 may be disposed at a bottom of the animal tray 200 and be slidably connected to the slide rail 1420. A count of sliders 1450 may be set to one or more (e.g., 2 or 4) based on the length of the animal tray. The animal tray 200 may be able to slide or move on the slide rail 1420 driven by various external forces (e.g., pushing or pulling by an operator).

The slidable connection between the animal tray 200 and the animal chamber 1400 may also be achieved in other manners. For example, two slide rails may be disposed at preset positions on both inner sidewalls of the chamber body 1410, and two sliders may correspondingly be disposed on both sides of the animal tray 200, each slidably connected to one of the two slide rails. This manner can improve the stability of the connection and placement between the animal tray 200 and the animal chamber 1400, as well as the stability of the animal tray during movement. As another example, a slide rail may be disposed at the bottom of the animal tray 200, and a slider capable of moving in that slide rail may be disposed at the bottom of the chamber body 1410.

In some embodiments of the present disclosure, providing a slide rail within the animal chamber enables stable movement of the animal tray along a preset direction and allows the imaging animal on the animal tray to maintain a fixed posture during movement, thereby enhancing the efficiency and accuracy of subsequent MR image stitching and avoiding reduced stitching accuracy due to changes in the posture of the imaging animal caused by a plurality of movements of the animal tray.

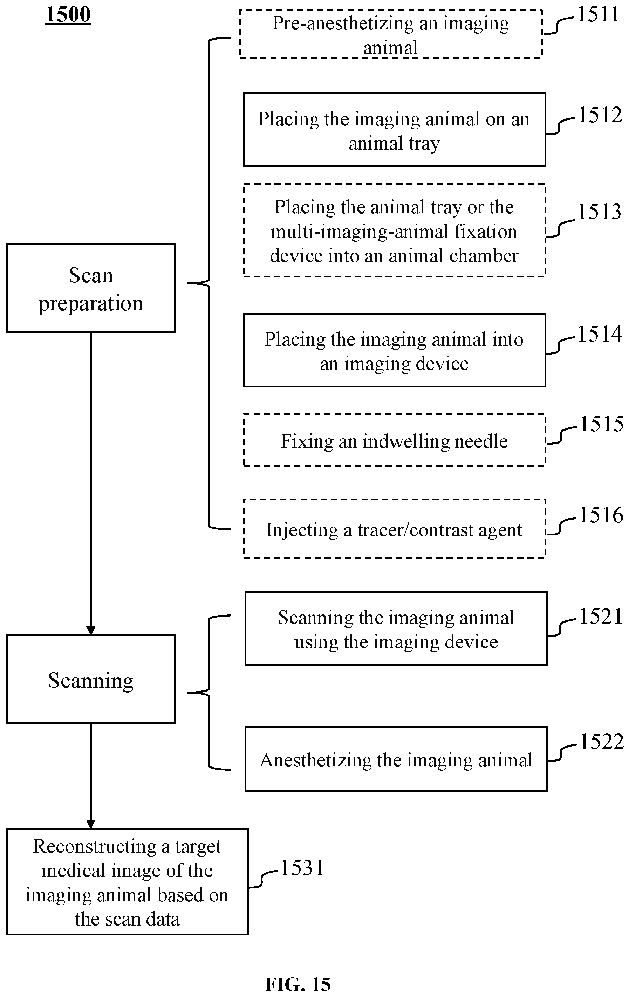

FIG. 15 is a flowchart illustrating an exemplary process for scanning an imaging animal according to some embodiments of the present disclosure. In some embodiments, one or more operations of process 1500 may be performed by the processing device 170.

As shown in FIG. 15, the process 1500 may include one or more of the following operations 1511 to 1516, which are performed during a scanning preparation stage.

In 1511, the imaging animal may be pre-anesthetized.

In some embodiments, before placing the imaging animal on the animal tray, the imaging animal may be pre-anesthetized to facilitate subsequent placement and fixation of the imaging animal onto the animal tray. In some embodiments, pre-anesthesia may be performed using an induction box. For example, the imaging animal may be placed in an induction box filled with anesthetic gas to achieve pre-anesthesia of the imaging animal.

In 1512, the imaging animal may be placed on an animal tray.

In some embodiments, the imaging animal may be placed on the animal tray in a preset positioning posture. The animal tray is provided with body part fixation portions for fixing the preset positioning posture of the imaging animal.

In some embodiments, an operator may select a suitable animal tray based on information such as a type and a body size of the imaging animal. The imaging animal may be fixed via the body part fixation portions of the animal tray (e.g., a head fixation portion, a forelimb fixation portion, a hindlimb fixation portion, and a tail fixation portion). The imaging animal, once fixed on the tray, maintains the preset positioning posture. More descriptions regarding the tray and the body part fixation portions may be found elsewhere in the present disclosure (e.g., FIG. 2).

In some embodiments, in a multi-animal experiment, each imaging animal may be individually placed on one animal tray. Subsequently, the plurality of animal trays are placed on a support portion of a multi-imaging-animal fixation device. The plurality of animal trays may be placed on the support portion via detachable connections (e.g., snap-fit connections or card slot connections). More descriptions regarding the support portion may be found elsewhere in the present disclosure (e.g., FIGs. 9A to 9C).

In some embodiments of the present disclosure, placing the imaging animal on the animal tray in the preset positioning posture fixes the posture of the imaging animal during scanning, which can help reduce motion artifacts and improve the quality of scanned images.

In 1513, the animal tray or the multi-imaging-animal fixation device may be placed into an animal chamber.

When there is only one imaging animal, the imaging animal may be placed into the animal chamber via the animal tray. When there are two or more imaging animals, the two or more imaging animals may be placed into the animal chamber via the multi-imaging-animal fixation device. In some embodiments of the present disclosure, placing the animal trays on the multi-imaging-animal fixation device and then placing the multi-imaging-animal fixation device into the animal chamber for scanning imaging makes experimental operations more convenient and helps improve experimental efficiency.

In 1514, the imaging animal may be placed into an imaging device.

In some embodiments, the imaging animal may be placed into the imaging device via one of the animal tray, the multi-imaging-animal fixation device, or the animal chamber, enabling the imaging device to scan the imaging animal. For example, the animal tray along with the imaging animal may be placed on a scanning bed and then sent into a scanning cavity via the scanning bed. In some embodiments, the animal chamber may be mounted on the scanning bed or be a part of the scanning bed. After placing the animal tray or the multi-imaging-animal fixation device into the animal chamber in 1513, the imaging animal may be sent into the scanning cavity by moving the scanning bed.

In 1515, an indwelling needle may be fixed.

In some scenarios, an indwelling needle needs to be placed in the tail of the imaging animal. In some embodiments, the indwelling needle may be fixed in a manner such as adhesive tape fixation or an external fixator. In some embodiments, the fixation effect of the tail fixation portion may be used to fix the indwelling needle. Due to the fixation effect of the tail fixation portion, the indwelling needle located in the tail of the imaging animal is also fixed, thereby preventing the indwelling needle from becoming ineffective due to a change in position.

In 1516, a tracer/contrast agent is injected.

The tracer is typically a radioactive substance used in scans such as PET or SPECT to help observe metabolic activity or physiological processes. For example, the tracer may include Fludeoxyglucose (FDG). The contrast agent is used to enhance the contrast in X-ray, CT, or MRI images and is typically a substance containing iodine or barium, primarily used to display anatomical structures. The contrast agent may include gadolinium diethylenetriamine pentaacetic acid, gadopentetate dimeglumine, or the like.

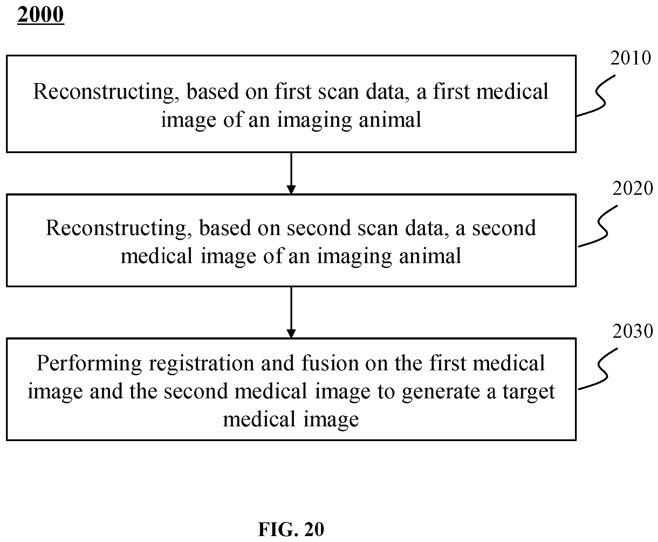

The injection of the tracer or contrast agent may be performed in various manners. For example, the user may use an external injection device (e.g., a vein fixation injector) to inject the tracer or contrast agent into the imaging animal. In some embodiments, the indwelling needle may be used to inject the tracer or contrast agent into each of the plurality of imaging animals. In other embodiments, the indwelling needle and the external injection device may be connected via an injection conduit, and the contrast agent or tracer may be injected into the imaging animal via the external injection device, the injection conduit, and the indwelling needle. More descriptions regarding the tracer or contrast agent may also be found elsewhere in the present disclosure (e.g., FIG. 20, etc.).

It should be noted that the above operations 1511 to 1516 are not intended to be limiting. One or more of the operations may be combined or omitted according to actual circumstances. For example, the operations 1511, 1513, 1515, and 1516 are optional.

The process 1500 may further include operations 1521 and 1522, which are performed during a scanning stage.

In 1521, the processing device 170 may scan the imaging animal using the imaging device 150 to acquire scan data of the imaging animal. During the scan, the imaging animal is placed on the animal tray in the preset positioning posture and is placed into the imaging device 150 via the animal tray. The animal tray includes body part fixation portions disposed at fixed positions on the animal tray and configured to maintain the preset positioning posture of the imaging animal.

The scan may be a single-modality scan or a multi-modality scan. When the scan is a multi-modality scan, the scan data includes scan data corresponding to each modality. In a multi-animal experiment, the scan data includes scan data for each imaging animal among the plurality of imaging animals.

In 1522, the imaging animal may be anesthetized.

In some embodiments, the animal tray includes an anesthesia interface disposed at a first end portion of the animal tray. During the scan, anesthetic gas is delivered to the imaging animal through the anesthesia interface to keep the imaging animal in an anesthetized state. In some embodiments, the anesthesia interface of the animal tray is in communication with an anesthesia pipeline. The anesthetic gas is delivered to the mouth and the nose of the imaging animal via the anesthesia pipeline and the anesthesia interface to anesthetize the imaging animal. The anesthetic gas may include ether, chloroform, isoflurane, or the like. More descriptions regarding the anesthesia interface may be found elsewhere in the present disclosure (e.g., FIG. 5).

In 1531, a target medical image of the imaging animal may be reconstructed based on the scan data.

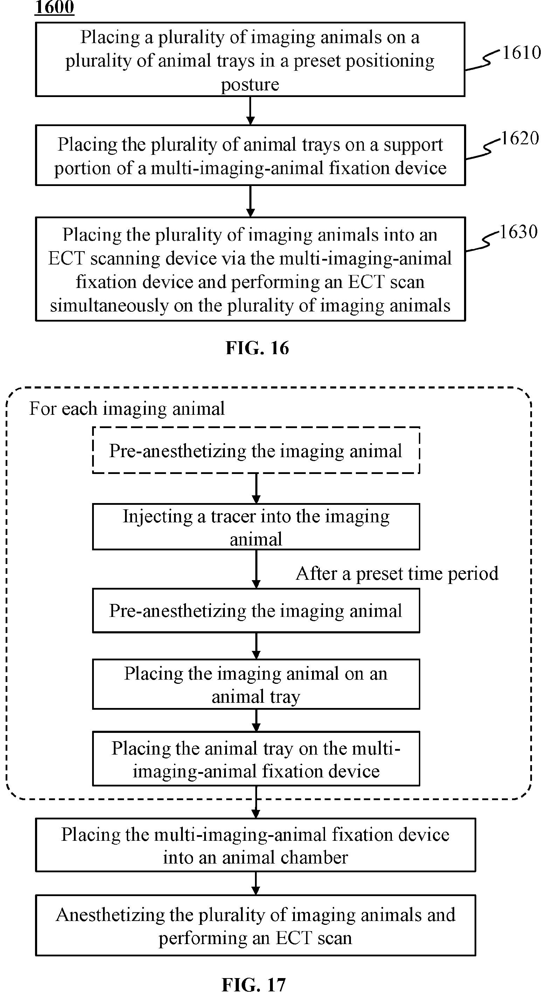

Upon completion of the scan, the processing device 170 may perform image reconstruction via an image reconstruction algorithm based on the scan data to obtain the target medical image. In some embodiments, when there are two or more imaging animals, the processing device 170 may reconstruct a target medical image for each imaging animal based on the scan data. As another example, when the imaging device 150 is a multi-modality scanning device, the processing device 170 may reconstruct images of different modalities and perform registration and fusion on the images of different modalities to generate the target medical image. More descriptions may be found in FIGs. 16 to 29 and descriptions thereof.

In some embodiments, the animal tray includes a physiological signal acquisition device configured to acquire a physiological signal of the imaging animal. The processing device 170 may perform gated reconstruction on the scan data based on the physiological signal to generate the target medical image. The gated reconstruction includes respiratory gated reconstruction and/or cardiac gated reconstruction, which may be performed based on various gated reconstruction algorithms. More descriptions regarding the physiological signal acquisition device may be found elsewhere in the present disclosure (e.g., FIG. 7).

In some embodiments, the imaging device is an MRI device, and each animal tray includes a receiving coil embedded in a bottom of the animal tray. The scan data includes MR data acquired by the receiving coil of each animal tray from the imaging animal placed on that animal tray. For each animal tray, the processing device 170 may generate the MR image (i.e., the target medical image) of the imaging animal placed on the animal tray based on the MR data acquired by the receiving coil of the animal tray. When there are a plurality of imaging animals, the processing device 170 may generate a plurality of MR images corresponding to the plurality of imaging animals.

FIG. 16 is a flowchart illustrating an exemplary method for animal emission computed tomography scanning according to some embodiments of the present disclosure.

In 1610, a plurality of imaging animals may be placed on a plurality of animal trays in a preset positioning posture.

In some embodiments, each of the plurality of imaging animals is placed on one animal tray. The preset positioning posture refers to the positioning posture that the imaging animal needs to hold during the scan. In some embodiments, the body part fixation portions of the animal tray may be configured to fix the positioning posture of the imaging animal, thereby maintaining the preset positioning posture of the imaging animal. More descriptions regarding the body part fixation portions may be found elsewhere in the present disclosure (e.g., FIG. 2).

In 1620, the plurality of animal trays may be placed on a support portion of a multi-imaging-animal fixation device.

In some embodiments, the plurality of animal trays corresponding to the plurality of imaging animals may be placed on the support portion of the multi-imaging-animal fixation device. The plurality of animal trays may be placed on the support portion via detachable connections (e.g., snap-fit connections or card slot connections). More descriptions regarding the connection between the animal trays and the support portion may be found in FIGs. 9A to 10B and related descriptions thereof.

In 1630, the plurality of imaging animals may be placed into an ECT scanning device via the multi-imaging-animal fixation device, and an ECT scan is performed simultaneously on the plurality of imaging animals.

In some embodiments, the plurality of imaging animals may be placed into the imaging device via the multi-imaging-animal fixation device. For example, the multi-imaging-animal fixation device may be placed inside an animal chamber via a detachable connection and then placed into the ECT scanning device via the animal chamber. More descriptions regarding the connection between the multi-imaging-animal fixation device and the animal chamber may be found in FIGs. 12 and 13 and related descriptions thereof.

In some embodiments, the process 1600 may further include: generating scan images of the plurality of imaging animals based on scan data acquired during the ECT scan. For example, PET images of the plurality of imaging animals may be generated based on PET scan data acquired during a PET scan. In some embodiments, when the ECT scan is a multi-modality scan, multi-modality scan data may be registered and fused to generate multi-modality fusion images of the plurality of imaging animals. For example, when performing PET/CT scan on the plurality of imaging animals, PET images of the plurality of imaging animals may be generated based on PET scan data, CT images of the plurality of imaging animals may be generated based on CT scan data, and the PET images and CT images of the plurality of imaging animals may be registered and fused to obtain PET/CT images of the plurality of imaging animals.

In some embodiments, during the ECT scan process, anesthetic gas may be delivered to the plurality of imaging animals via anesthesia interfaces to anesthetize the plurality of imaging animals.

In animal experiments, due to different experimental requirements, ECT scans of the imaging animals may include a static ECT scan and a dynamic ECT scan.

FIG. 17 is a flowchart illustrating an exemplary static ECT scan process according to some embodiments of the present disclosure. As shown in FIG. 17, when performing a static ECT scan, the following operations may be performed for each imaging animal: a tracer may be injected into the imaging animal; after a preset time period (e.g., 2 h, 5 h, or 7 h) following the tracer injection, the imaging animal may be pre-anesthetized; the imaging animal after the pre-anesthesia treatment may be placed on an animal tray in the preset positioning posture; and the animal tray may be placed on the multi-imaging-animal fixation device. After all animal trays carrying imaging animals are placed on the multi-imaging-animal fixation device, the multi-imaging-animal fixation device may be placed in an animal chamber, and the plurality of imaging animals may be anesthetized and undergo an ECT scan. More descriptions regarding pre-anesthesia and anesthesia may also be found elsewhere in the present disclosure (e.g., FIG. 16).

In some embodiments, the tracer may include any marker detectable in ECT. If a user is not proficient in performing tracer injection on imaging animals, before injecting the tracer, the plurality of imaging animals may be placed in an induction box and a pre-anesthesia treatment may be performed on the plurality of imaging animals; then the tracer may be injected into the plurality of imaging animals after the pre-anesthesia treatment.

In some embodiments of the present disclosure, by performing pre-anesthesia treatment on the imaging animals first and then injecting the tracer, the injection of the tracer becomes more convenient, which can help improve experimental efficiency for users who are not proficient in the operation.

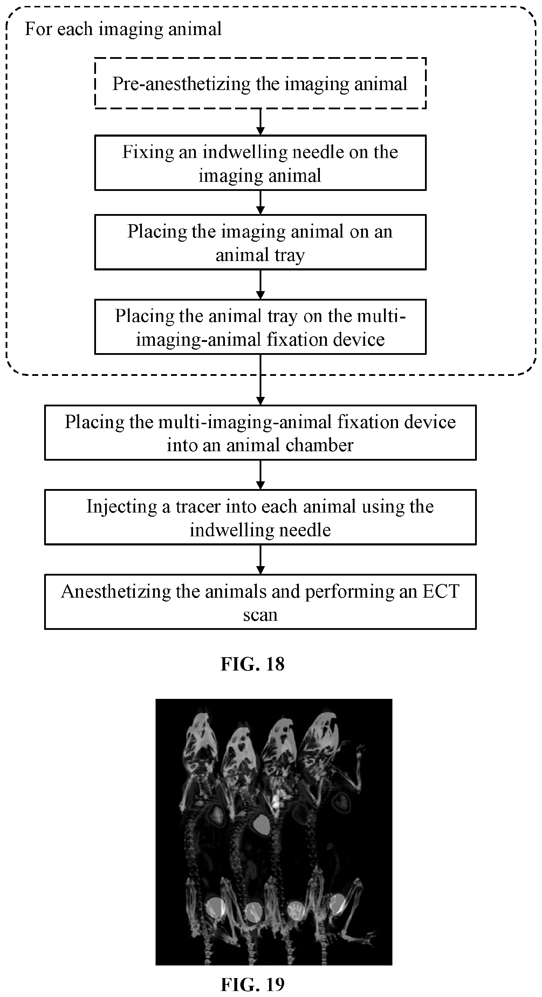

FIG. 18 is a flowchart illustrating an exemplary dynamic ECT scan process according to some embodiments of the present disclosure.

As shown in FIG. 18, when performing a dynamic ECT scan, the following operations may be performed for each imaging animal: the imaging animal may be pre-anesthetized; an indwelling needle may be fixed on the imaging animal after the pre-anesthesia treatment; the imaging animal after the pre-anesthesia treatment and the fixation of the indwelling needle may be placed on the animal tray in the preset positioning posture; and the animal tray may be placed on the multi-imaging-animal fixation device. After the animal trays carrying the imaging animals are placed on the multi-imaging-animal fixation device, the multi-imaging-animal fixation device may be placed in an animal chamber. The tracer may be injected into each of the plurality of imaging animals using the indwelling needle, and the plurality of imaging animals may be anesthetized and undergo an ECT scan. In some embodiments, after the tracer is injected into the imaging animals, anesthesia and scanning operations may be performed on the imaging animals immediately.

In some embodiments, first modality scan data may be acquired by simultaneously performing an ECT scan on the plurality of imaging animals via a scanning device; then second modality scan data may be acquired by performing a CT scan or an MR scan on the plurality of imaging animals; further, the second modality scan data and the first modality scan data may be registered and fused. The first modality scan data refers to image data obtained based on the ECT scan. The second modality scan data refers to image data obtained based on the CT scan or MR scan.

In some embodiments, a processing device may perform registration and fusion on the second modality scan data and the first modality scan data using any suitable image fusion algorithm. In some embodiments, the registration and fusion may be performed based on marker accommodation portions disposed on the animal tray. Merely by way of example, FIG. 19 is an image illustrating an exemplary result of registration and fusion based on first modality scan data and second modality scan data according to some embodiments of the present disclosure. As shown in FIG. 19, the registered and fused image can not only accurately display the tissue structure and organ metabolic activity of the imaging animal but also has fewer motion artifacts and higher image quality.

In some embodiments of the present disclosure, by performing a multi-modality scan on the imaging animal via a scanning device and registering and fusing scan data from different imaging modalities, a fused image that simultaneously displays the anatomical structure and organ metabolic activity of the imaging animal can be obtained, providing more data support for experimental analysis.

FIG. 20 is a flowchart illustrating an exemplary process for generating a target medical image according to some embodiments of the present disclosure. In some embodiments, the imaging device in operation 1522 includes a first imaging device and a second imaging device, and the acquired scan data includes first scan data acquired by the first imaging device and second scan data acquired by the second imaging device. The target medical image may be generated through process 2000 shown in FIG. 20.

In 2010, a first medical image of the imaging animal may be reconstructed based on the first scan data.

The first scan data refers to scan data of the imaging animal acquired by the first scanning device during a first scan.

In 2020, a second medical image of the imaging animal may be reconstructed based on the second scan data.

The second scan data refers to scan data of the imaging animal acquired by the second scanning device during a second scan.

In some embodiments, the first scan and the second scan are scans of different modalities. In some embodiments, one of the first scan and the second scan is an ECT scan. In some embodiments, the other of the first scan and the second scan is an MR scan. It should be understood that since the imaging modalities of the first scan and the second scan are different, the modalities of the first scanning device and the second scanning device are also different. For example, if the first scan is an ECT scan, the first scanning device may include a PET scanning device, a SPECT scanning device, a PET/CT scanning device, a PET/MR scanning device, a SPECT/CT scanning device, a SPECT/MR scanning device, etc.; if the second scan is an MR scan, the second scanning device may include an MRI device. In some embodiments, the first scanning device and the second scanning device may be two independent scanning devices with different modalities.

In some embodiments, the first scan and the second scan may be scans of the same modality, but the first scanning device and the second scanning device are two different independent scanning devices. For example, both the first scan and the second scan are PET scans, but the first scanning device and the second scanning device are two different PET scanning devices. Merely by way of example, the first medical image and the second medical image are PET images of the imaging animal acquired by two different PET scanning devices at two different times.

During the first scan and the second scan, the imaging animal is placed on the animal tray in the preset positioning posture and is placed into the first scanning device and the second scanning device via the animal tray. The preset positioning posture refers to the positioning posture during the scan of the imaging animal. In some embodiments, the body part fixation portions of the animal tray may be utilized to fix the posture of the imaging animal, so that the imaging animal can maintain the same preset positioning posture in the first scan and the second scan.

In some embodiments, the first scanning device may include a first animal chamber. During the first scan, the animal tray carrying the imaging animal may be placed in the first animal chamber. The second scanning device may include a second animal chamber. During the second scan, the animal tray carrying the imaging animal may be placed in the second animal chamber. The first animal chamber and the second animal chamber are similar to the animal chamber 130 described in FIG. 1 and will not be elaborated here. In some embodiments, the same animal chamber may be used to place the animal tray of the imaging animal into the scanning cavities of the first scanning device and the second scanning device successively. The animal chamber may be mounted onto scanning beds of the first scanning device and the second scanning device, respectively.

In some embodiments, during the first scan and the second scan, anesthetic gas may be delivered to the imaging animal via an anesthesia interface to anesthetize the imaging animal. In some embodiments, before placing the imaging animal on the animal tray, the imaging animal may be pre-anesthetized. More descriptions regarding the anesthesia treatment and the pre-anesthesia may be found elsewhere in the present disclosure (e.g., FIG. 15).

In some embodiments, if one of the first scan and the second scan is an ECT scan, before performing the ECT scan, a tracer may be injected into the imaging animal. Merely by way of example, a tracer may first be injected into the imaging animal; after a preset time period (e.g., 2 h, 5 h, 7 h, etc.) following the tracer injection, the imaging animal may be pre-anesthetized (e.g., pre-anesthesia treatment performed via an induction box); then the imaging animal may be placed on the animal tray. More descriptions regarding the tracer and pre-anesthesia may also be found elsewhere in the present disclosure (e.g., FIGs. 16 and 17).

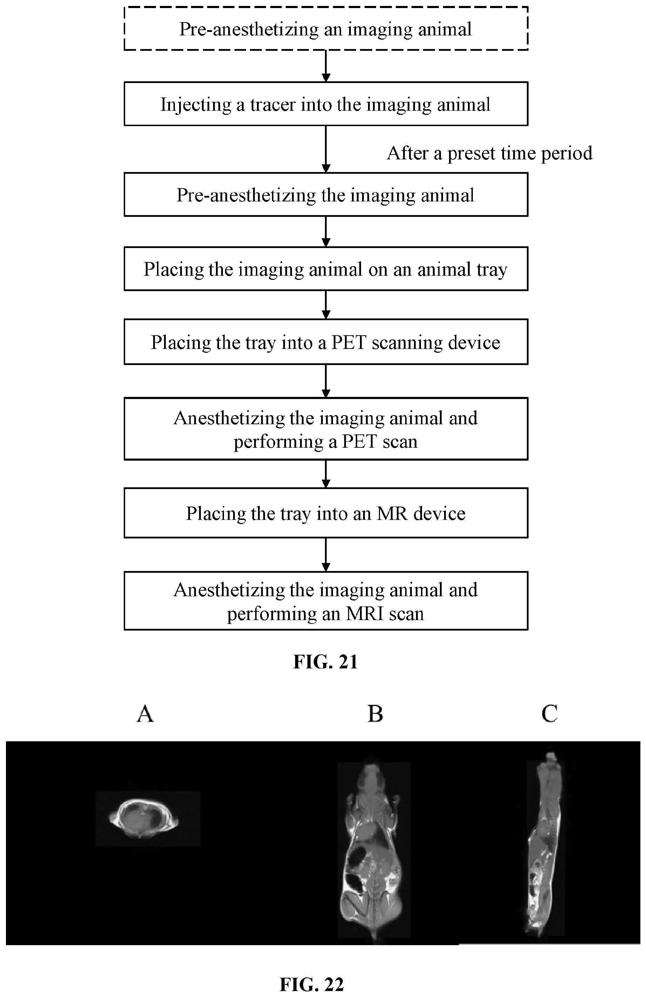

Merely by way of example, the following description uses a PET scan as an exemplary first scan and an MR scan as an exemplary second scan. FIG. 21 is a flowchart illustrating an exemplary PET scanning and MR scanning process according to some embodiments of the present disclosure. As shown in FIG. 21, when performing a PET scan and an MR scan on an imaging animal, the following operations may be performed on the imaging animal: a tracer may be injected into the imaging animal; after a preset time period (e.g., 2 h, 5 h, 7 h, etc.) following the tracer injection, the imaging animal may be pre-anesthetized; the imaging animal after the pre-anesthesia treatment may be placed on an animal tray in the preset positioning posture; the animal tray may be placed into a PET scanning device (e.g., placed into an animal chamber of the PET scanning device), and the imaging animal may be anesthetized and undergo the PET scan; then the animal tray may be placed into an MRI device (e.g., placed into an animal chamber of the MRI device), and the imaging animal may be anesthetized and undergo the MR scan. In some embodiments, before performing the MR scan, a receiving coil may be installed on an exterior of the animal chamber of the MRI device. In some embodiments, a receiving coil (e.g., the receiving coil 720) is embedded in the animal tray, in which case installation of the receiving coil is not required.

It should be understood that the process shown in FIG. 21 is for illustrative purposes only. In some embodiments, the MR scan may be performed first, followed by the PET scan. In some embodiments, the first scan and the second scan may be any other type of scans.

In some embodiments, the animal tray is provided with one or more marker accommodation portions configured to accommodate a marker detectable in the first medical image and the second medical image, for performing subsequent registration and fusion. The marker accommodation portion(s) may be fixedly installed or detachably installed on the animal tray. For example, before placing the imaging animal on the animal tray, the marker may be injected into the marker accommodation portion(s), and the marker accommodation portion(s) may be installed on the animal tray. In some embodiments, the marker accommodation portion(s) may be detachably mounted onto the animal tray via fixing structure(s) on the animal tray. More descriptions regarding the fixing structure(s) may be found in FIG. 3 and related descriptions thereof. More descriptions regarding the marker accommodation portion(s) may be found in FIGs. 2, 4A to 4B and related descriptions thereof.

In 2030, registration and fusion may be performed on the first medical image and the second medical image to generate a target medical image of the imaging animal.

The target medical image refers to the registered and fused medical image. For example, the target medical image may be a PET/MR image. In some embodiments, any suitable image registration algorithm and image fusion algorithm may be used to perform registration and fusion on the first medical image and the second medical image to generate the target medical image. In some embodiments, the registration and fusion may be performed based on the marker in the marker accommodation portion(s) disposed on the animal tray. For example, image registration may be performed based on the marker in the first medical image and the second medical image to generate a registered first medical image and a registered second medical image, and the registered first medical image and the registered second medical image may be fused to obtain the target medical image.

Merely by way of example, FIG. 22 is an image illustrating an exemplary result of fusion of a PET scan image and an MR scan image according to some embodiments of the present disclosure. As shown in FIG. 22, an image A, an image B, and an image C are fused images in the axial, coronal, and sagittal planes of the imaging animal, respectively. It may be seen from the images that the registered and fused images can not only accurately display the tissue structure and organ metabolic activity of the imaging animal but also have fewer motion artifacts and higher image quality.

In some embodiments of the present disclosure, by placing the imaging animal on the animal tray in the preset positioning posture, the posture of the imaging animal during scanning can be fixed, ensuring consistent body posture across different scanning devices, which can help reduce motion artifacts and improve the quality of scanned images. By performing multi-modality scanning imaging on the imaging animal using the first scanning device and the second scanning device, and registering and fusing scan data from different imaging modalities via the marker in the marker accommodation portion(s), the speed and accuracy of registration and fusion can be improved, and a fused image that simultaneously displays the anatomical structure and organ metabolic activity of the imaging animal can be obtained, providing more data support for experimental analysis.

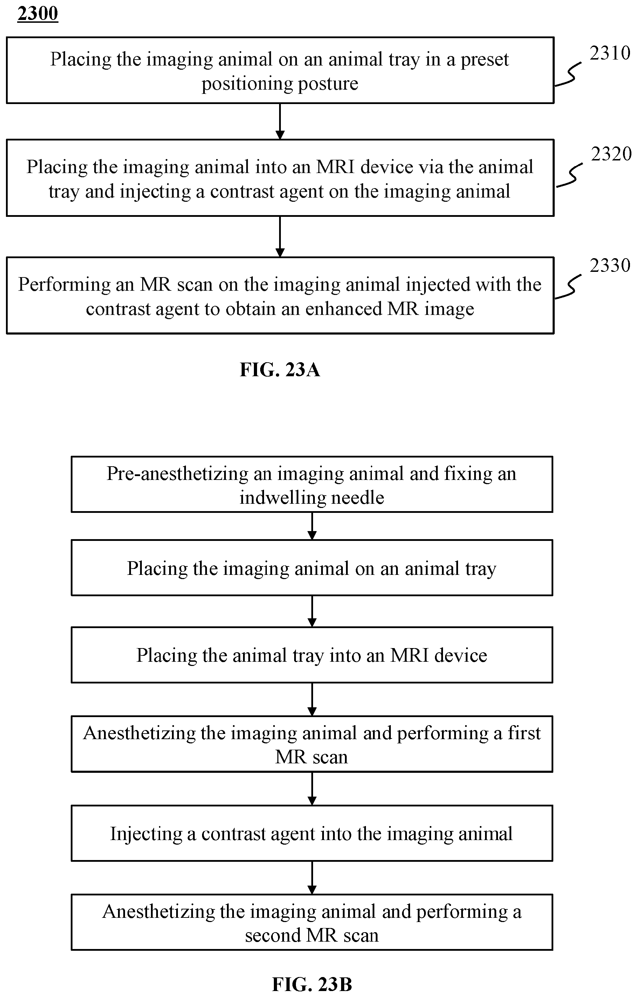

FIG. 23A is a flowchart illustrating an exemplary process for an animal enhanced MR scan according to some embodiments of the present disclosure. In some embodiments, one or more operations of process 2300 may be performed by the processing device 170.

In 2310, an imaging animal may be placed on an animal tray in a preset positioning posture.

In some embodiments, body part fixation portions of the animal tray may be configured to fix the posture of the imaging animal, thereby maintaining the preset positioning posture of the imaging animal. More descriptions regarding the body part fixation portions may be found in FIG. 3 and related descriptions thereof.

In some embodiments, before placing the imaging animal on the animal tray, the imaging animal may be pre-anesthetized. Further, an indwelling needle may be fixed on the imaging animal after the pre-anesthesia treatment. Then, the imaging animal after the pre-anesthesia treatment and the fixation of the indwelling needle may be placed on the animal tray in the preset positioning posture. In some embodiments, the imaging animal after the pre-anesthesia treatment may be placed on the animal tray in the preset positioning posture first, and then the indwelling needle may be fixed on the imaging animal on the animal tray.

In 2320, the imaging animal may be placed into an MRI device via the animal tray, and a contrast agent is injected into the imaging animal.

In some embodiments, the contrast agent may be injected into the imaging animal via the indwelling needle. In some embodiments, the MRI device includes an animal chamber. During the MR scanning process, the animal tray carrying the imaging animal may be placed in the animal chamber.

In 2330, the imaging animal injected with the contrast agent may be scanned using the MRI device to obtain an enhanced MR image.

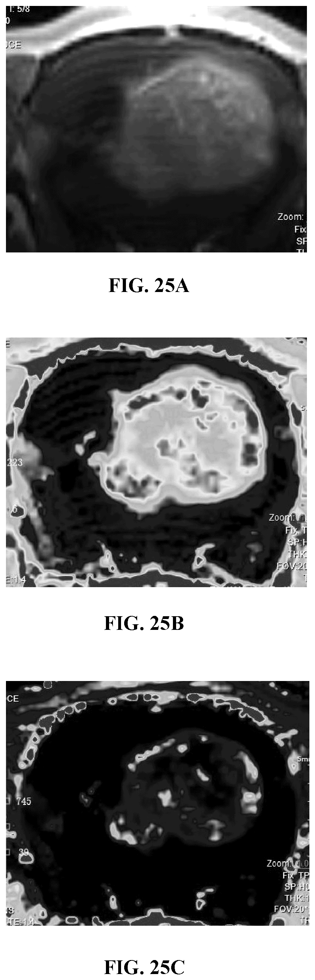

The enhanced MR image refers to an MR image whose observation effect is enhanced by the contrast agent. Merely by way of example, FIG. 25A is a schematic diagram illustrating an enhanced MR image of a brain tumor of an imaging animal according to some embodiments of the present disclosure. As shown in FIG. 25A, the enhanced MR image has higher tissue contrast, clearly reflecting the structure of the brain tumor region and the tumor margin region.

In some embodiments, the processing device 170 may obtain scan data acquired during the MR scan and reconstruct the enhanced MR image of the imaging animal based on the scan data. In some embodiments, the MRI device may perform an MR scan on the imaging animal at fixed time intervals (e.g., 1 minute) to obtain a plurality of enhanced MR images across different time periods. In some embodiments, the processing device 170 may perform data analysis on the enhanced MR images corresponding to the different time periods. Merely by way of example, FIG. 25B is a schematic diagram illustrating a gadolinium concentration map in the intercellular space of an imaging animal according to some embodiments of the present disclosure; FIG. 25C is a schematic diagram illustrating a gadolinium volume transfer rate map of an imaging animal according to some embodiments of the present disclosure. From FIGs. 25B and 25C, the gadolinium concentration distribution in the intercellular space and the gadolinium volume transfer rate can be analyzed, providing more data support for subsequent experiments.

In some embodiments, during the MR scanning process, anesthetic gas may be delivered to the imaging animal via an anesthesia interface to anesthetize the imaging animal. In some embodiments, before injecting the contrast agent into the imaging animal, an initial MR image may be obtained by scanning the imaging animal using the MRI device. The initial MR image refers to an image obtained by scanning without injecting the contrast agent. In some embodiments, comparative analysis may be performed on the initial MR image and the enhanced MR image.

For illustrative purposes, the following description takes as an example the scenario where an MR scan without contrast agent injection (also referred to as a first MR scan) is performed on the imaging animal first, followed by an enhanced MR scan with contrast agent injection (also referred to as a second MR scan).

FIG. 23B is a flowchart illustrating an exemplary process for performing an MR scan and an enhanced MR scan on an imaging animal, according to some embodiments of the present disclosure.

As shown in FIG. 23B, when performing the MR scan and the enhanced MR scan on the imaging animal, the following operations may be performed on the imaging animal. Pre-anesthesia treatment and fixation of an indwelling needle may be performed on the imaging animal. The imaging animal after the pre-anesthesia treatment and the fixation of the indwelling needle is placed on an animal tray in the preset positioning posture. The imaging animal is placed into the MRI device via the animal tray, e.g., placed into an animal chamber or on a scanning bed of the MRI device. The imaging animal is anesthetized and undergoes a first MR scan to obtain an initial MR image. In some embodiments, the animal tray and the imaging animal placed thereon are moved into a scanning cavity of the MRI device by moving the scanning bed, the imaging animal is anesthetized using the anesthesia interface on the animal tray, and the MRI device is controlled to perform the first MR scan. A contrast agent is injected into the imaging animal. The imaging animal is anesthetized and undergoes a second MR scan to obtain an enhanced MR image. In some embodiments, the animal tray and the imaging animal placed thereon are moved again into the scanning cavity of the MRI device by moving the scanning bed, and the imaging animal is anesthetized and undergoes the second MR scan.

In some embodiments of the present disclosure, injecting a contrast agent into the imaging animal and performing the MR scan to obtain an enhanced MR image can increase tissue contrast in MR images and display the structure of the tumor region and tumor margin region more clearly, providing more data support for animal experiments.

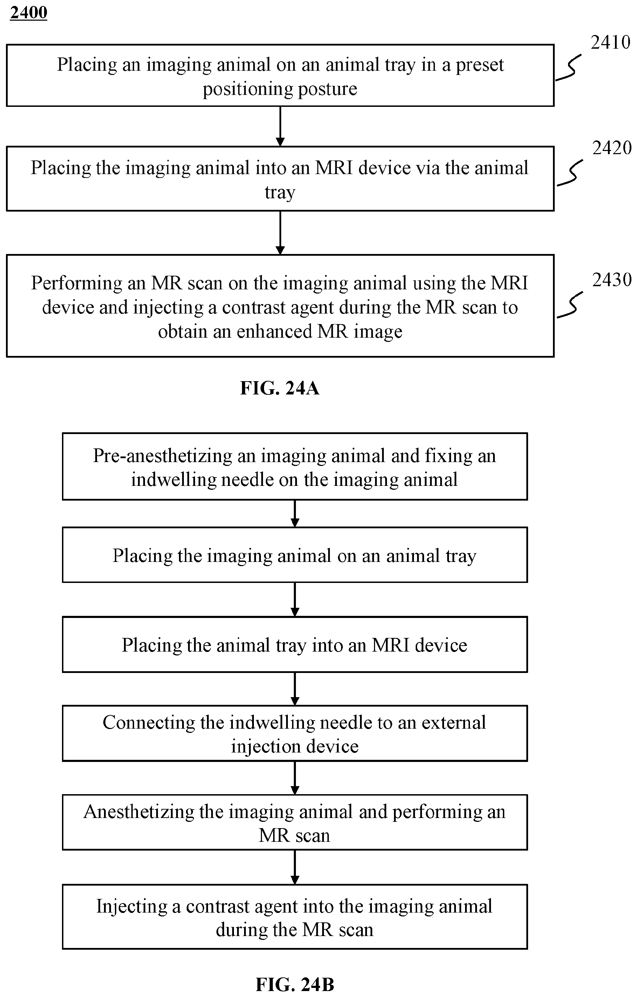

FIG. 24A is a flowchart illustrating another exemplary process for an animal enhanced MR scan according to some embodiments of the present disclosure. As shown in FIG. 24A, process 2400 may include the following operations.

In 2410, the imaging animal may be placed on an animal tray in a preset positioning posture. Operation 2410 is similar to operation 2310. In some embodiments, the imaging animal may be placed in an induction box for pre-anesthesia treatment, an indwelling needle may be fixed on the imaging animal after the pre-anesthesia treatment, and then the imaging animal after the pre-anesthesia treatment and the fixation of the indwelling needle may be placed on the animal tray in the preset positioning posture. More descriptions regarding the foregoing embodiments may be found in operation 2310 and related descriptions thereof.

In 2420, the imaging animal may be placed into the MRI device via the animal tray. More descriptions regarding placing the imaging animal into the MRI device via the animal tray may be found in operation 2320.

In 2430, an MR scan is performed on the imaging animal using the MRI device, and the contrast agent is injected into the imaging animal during the MR scan to obtain an enhanced MR image.

In some embodiments, before the MR scan, the indwelling needle may be connected to an external injection device. During the MR scan, the contrast agent is injected into the imaging animal via the external injection device and the indwelling needle. In some embodiments, before injecting the contrast agent into the imaging animal, an initial MR image may be obtained by scanning the imaging animal using the MRI device. In some embodiments, comparative analysis may be performed on the initial MR image and the enhanced MR image. More descriptions regarding the foregoing embodiments may be found in operation 2330 and related descriptions thereof.

Merely by way of example, the following description takes performing an MR scan without injecting a contrast agent on the imaging animal first, followed by an enhanced MR scan with the contrast agent injected as an example. FIG. 24B is a flowchart illustrating another exemplary process for performing an MR scan and an enhanced MR scan on an imaging animal, according to some embodiments of the present disclosure. As shown in FIG. 24B, when performing the MR scan and the enhanced MR scan on the imaging animal, the following operations may be performed on the imaging animal. A pre-anesthesia treatment and fixation of an indwelling needle are performed on the imaging animal. The imaging animal after the pre-anesthesia treatment and the fixation of the indwelling needle is placed on an animal tray in the preset positioning posture. The imaging animal is placed into the MRI device via the animal tray, e.g., placed into an animal chamber or on a scanning bed of the MRI device. The indwelling needle is connected to the external injection device. The imaging animal is anesthetized and undergoes the MR scan to obtain an initial MR image. Then, during the MR scan, the contrast agent is injected into the imaging animal to obtain an enhanced MR image. In some embodiments, the external injection device is turned on, and the contrast agent is injected into the imaging animal during the MR scan.

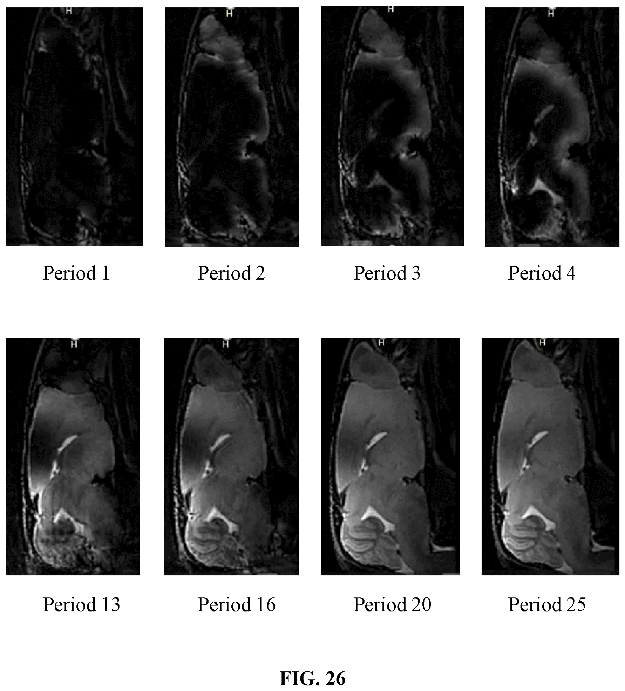

Merely by way of example, FIG. 26 is a schematic diagram illustrating exemplary enhanced MR images at different time periods according to some embodiments of the present disclosure. As shown in FIG. 26, across different time periods (e.g., period 1 to period 4, period 13 to period 25), as the amount of injected contrast agent increases and the contrast agent diffuses within the imaging animal, the tissue contrast and structural clarity in the enhanced MR images gradually improve.

In some embodiments of the present disclosure, connecting the indwelling needle to the external injection device enables injection of the contrast agent into the imaging animal during the MR scanning process, which can help simplify animal experimental operations and can also satisfy different experimental requirements.

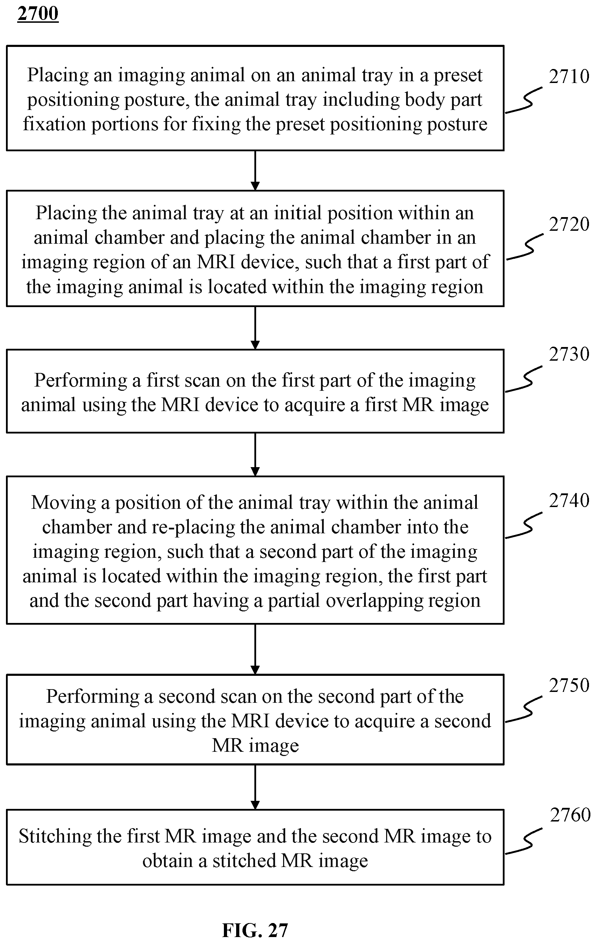

FIG. 27 is a flowchart illustrating an exemplary process of animal MRI according to some embodiments of the present disclosure. In some embodiments, one or more operations of process 2700 may be performed by the processing device 170.

In 2710, an imaging animal may be placed on an animal tray in a preset positioning posture. The animal tray includes body part fixation portions for fixing the preset positioning posture of the imaging animal.

In 2720, the animal tray may be placed at an initial position within an animal chamber, and the animal chamber is placed in an imaging region of an MRI device, such that a first part of the imaging animal is located within the imaging region.

The first part refers to a preset body part of the imaging animal, which may represent a body part corresponding to a first segment scan in multi-segment scans performed on the imaging animal using the MRI device. For example, the first part may be the head, chest, abdomen, or buttocks of the imaging animal. In some embodiments, the first part may be the upper body part of the imaging animal in a whole-body scan.

The initial position refers to a position within the animal chamber where the first part of the imaging animal may be imaged by the MRI device. When the animal tray is placed at the initial position within the animal chamber, the entirety of the animal tray is located within a chamber body of the animal chamber. For example, the initial position may represent that when the animal tray is fully placed within the animal chamber, a center point of the animal tray coincides with a center point of the animal chamber, or a distance between a first end of the animal tray and one end of the animal chamber reaches a preset distance threshold (e.g., 5 cm).

In some embodiments, before placing the animal chamber at the initial position, an operator may install an MR coil on an exterior of the animal chamber. The MR coil may be secured via a coil mounting structure provided on the animal chamber.

In some embodiments, the initial position may also be determined based on an installation position of the MR coil. For example, the initial position is a position that makes a center point of the first part (e.g., the chest position) to be aligned with the installation position of the MR coil. The operator may determine a current scale reading indicated by an indication mark based on the indication mark of the animal chamber and a scale indicator device of the animal tray, thereby determining whether the animal tray is at the initial position. The imaging region refers to a scanning region of the MRI device.

More descriptions regarding the animal tray may be found elsewhere in the present disclosure (e.g., FIGs. 2 or 3). More descriptions regarding the animal chamber, the indication mark, and the scale indicator device may be found in FIG. 14 and descriptions thereof.

In 2730, a first scan may be performed on the first part of the imaging animal using the MRI device to acquire a first MR image.

In some embodiments, in response to determining that the first part of the imaging animal is located within the imaging region, the processing device may control the MRI device to scan the imaging animal, wherein this scan is referred to as the first scan. The processing device 170 may control the MRI device to perform the first scan on the first part of the imaging animal according to a preset scanning protocol (e.g., a scanning duration).

The first MR image refers to a scan image acquired by the first scan. The first MR image may include one or more MR images presenting the internal structure corresponding to the first part. In some embodiments, the processing device may perform image processing on the first MR image. For example, the processing device may perform denoising processing and recognition processing on the first MR image according to preset algorithms (e.g., image denoising algorithms and image recognition algorithms).

In some embodiments, before the first scan, the processing device may deliver anesthetic gas to the imaging animal by an anesthesia interface to anesthetize the imaging animal, which ensures that the imaging animal maintains the preset positioning posture during the first scan. For example, the anesthesia interface may be externally connected to an anesthesia channel or anesthesia catheter, and anesthetic gas may be continuously delivered to the imaging animal via the anesthesia channel or anesthesia catheter and the anesthesia interface.

In 2740, a position of the animal tray within the animal chamber is moved, and the animal chamber may be re-placed into the imaging region such that a second part of the imaging animal is located within the imaging region.

The position after moving the animal tray may be referred to as a moved position. The second part is another body part having a partial overlapping region with the first part. In some embodiments, the second part may be the lower body part of the imaging animal in a whole-body scan.

In some embodiments, the operator may determine whether the current animal tray is at a desired position based on the scale indicator device of the animal tray and the indication mark of the animal chamber. For example, after performing the first scan, the operator may move the animal tray, and the animal tray slides along a longitudinal axis of the animal tray on a slide rail of the animal chamber, so that after the animal tray is moved, the second part of the imaging animal is located within the imaging region. In some embodiments, the operator may observe a real-time scale reading of the scale indicator device to determine whether the second part is aligned with the center of the MR coil. If so, the second scan may be performed.

In some embodiments, after any segment scan (e.g., the first scan or the second scan) ends and before moving the animal tray into the animal chamber, the operator may move the animal chamber out of the imaging region and remove the MR coil; and before re-placing the animal chamber into the imaging region, the operator re-install the MR coil on the exterior of the animal chamber.

It should be noted that when the animal chamber is in the imaging region and the MR coil is installed on the exterior of the animal chamber, the movement of the animal tray may be hindered by the constraints of the MRI scanning channel and the MR coil, making it difficult for the operator to adjust the animal tray precisely. Therefore, it is necessary to move the animal chamber out of the imaging region and remove the MR coil. This facilitates the operator's access to the animal tray and improves the accuracy of its repositioning.

In 2750, a second scan may be performed on the second part of the imaging animal using the MRI device to acquire a second MR image.

In some embodiments, in response to determining that the second part of the imaging animal is located within the imaging region, the processing device may control the MRI device to scan the imaging animal, and this scan is referred to as the second scan. The processing device may control the MRI device to perform the second scan on the second part of the imaging animal according to a preset scanning protocol.

The second MR image refers to a scan image corresponding to the second part of the imaging animal acquired by the second scan. The second MR image may include one or more MR images presenting the internal structure corresponding to the second part. In some embodiments, the processing device may perform image processing on the second MR image, e.g., denoising processing, recognition processing, etc., on the second MR image according to preset algorithms (e.g., image denoising algorithms or image recognition algorithms).

In some embodiments, the animal tray is provided with one or more marker accommodation portions, and each marker accommodation portion is configured for accommodating a marker detectable in an MRI modality. At least one marker accommodation portion of the one or more marker accommodation portions is located within the imaging region during both the first scan and the second scan. That is, at least one marker accommodation portion will appear in both the first MR image and the second MR image. More descriptions regarding the marker accommodation portions and the marker may be found elsewhere in the present disclosure (e.g., FIG. 2 and FIGs. 4A to 4B).

In 2760, the first MR image and the second MR image may be stitched to obtain a stitched MR image.

The stitched MR image is an MR image of the first part and the second part of the imaging animal after registration or alignment, which can present information about the internal structure of both the first part and the second part of the imaging animal. In some embodiments, the stitching may be performed based on an overlapping region between the first part and the second part. For example, the first MR image and the second MR image may be stitched based on the overlapping region displayed in the first MR image and the overlapping region displayed in the second MR image.

In some embodiments, as mentioned above, at least one marker accommodation portion appears in both the first MR image and the second MR image. The stitching may be performed based on the at least one marker accommodation portion displayed in the first MR image and the at least one marker accommodation portion displayed in the second MR image. For example, portions corresponding to the same scanning area in the two images may be aligned based on the at least one marker accommodation portion displayed in the first MR image and the at least one marker accommodation portion displayed in the second MR image, thereby achieving image stitching.

It should be noted that the first part and the second part are merely provided as examples. Operations similar to 2740 and 2750 above may be repeated to perform an n^{th} segment scan (e.g., a third scan, a fourth scan) on other parts of the imaging animal (e.g., a third part and a fourth part), thereby obtaining a corresponding n-th MR image (e.g., a third MR image and a fourth MR image). Based on the markers, the first MR image, the second MR image, and the n-th MR images may be stitched to obtain the stitched MR image.

In some embodiments, the first part of the imaging animal is one of the upper body and the lower body of the imaging animal, the second part is the other of the upper body and the lower body of the imaging animal, and the stitched MR image is a whole-body image of the imaging animal.

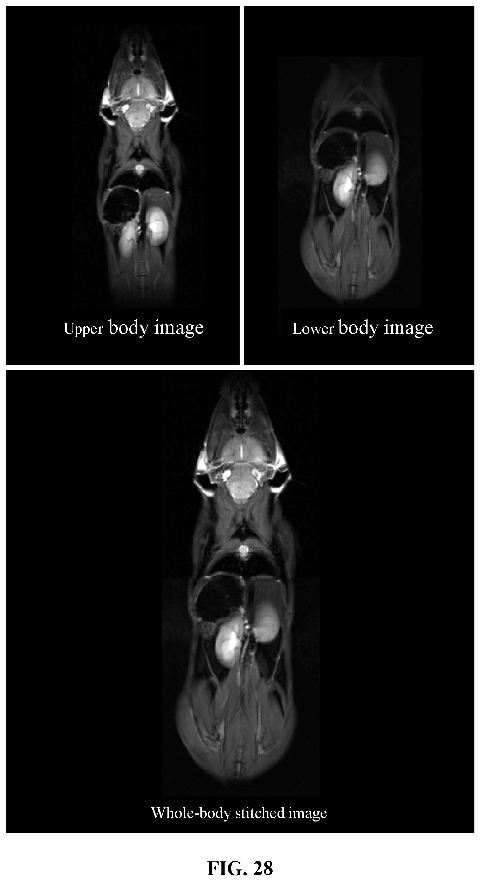

FIG. 28 is a schematic diagram illustrating an exemplary stitched MR image of a mouse according to some embodiments of the present disclosure.

As shown in FIG. 28, a first MR image 2810 is an MR image corresponding to the upper body of the mouse, which presents the internal structure of the upper body of the mouse. A second MR image 2820 is an MR image corresponding to the lower body of the mouse, which presents the internal structure of the lower body of the mouse. A stitched MR image 2830 is the stitching result of the first MR image 2810 and the second MR image 2820, which presents the internal structure of the upper body and the lower body of the mouse, i.e., the internal structure of the whole body of the mouse.

In some embodiments of the present disclosure, by providing marker accommodation portions, precise positional references can be provided for stitching MR images (e.g., the first MR image and the second MR image) corresponding to various parts (e.g., the first part, the second part) of the imaging animal, thereby making the obtained stitched MR image more accurate and avoiding errors or image misalignment that may occur when performing stitching by identifying parts of the imaging animal.

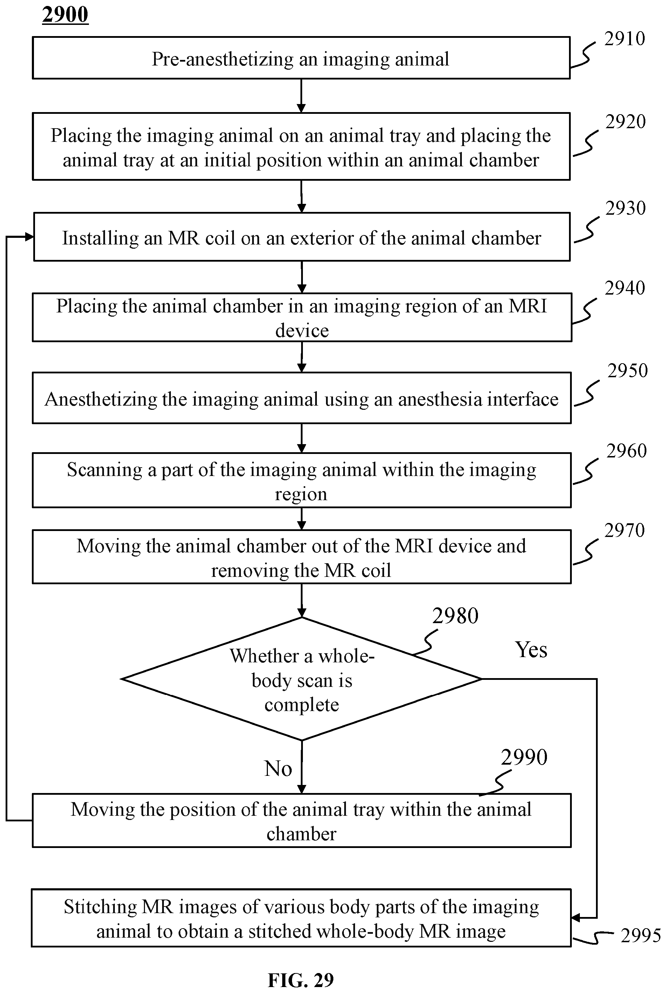

FIG. 29 is a flowchart illustrating another exemplary process of animal MRI according to some embodiments of the present disclosure. In some embodiments, one or more operations of process 2900 may be performed by the processing device 170.

In 2910, an imaging animal may be pre-anesthetized.

In some embodiments, an operator pre-anesthetizes the imaging animal to facilitate subsequent placement and fixation of the imaging animal onto the animal tray.

In 2920, the imaging animal may be placed on an animal tray, and the animal tray is placed at an initial position within an animal chamber.

In 2930, an MR coil may be installed on an exterior of the animal chamber.

The animal chamber may include one or more preset coil mounting structures for installing the MR coil.

In 2940, the animal chamber may be placed in an imaging region of an MRI device.

Before the animal chamber enters a scanning cavity of the MRI device, the animal chamber may be placed on a scanning bed of the MRI device, and the animal chamber may be placed into the imaging region by moving the scanning bed.

In 2950, the imaging animal may be anesthetized using an anesthesia interface.

In some embodiments, operation 2950 may be performed continuously throughout the scanning process. In some embodiments, operation 2950 may be performed before operation 2940.

In 2960, a part of the imaging animal within the imaging region may be scanned.

In 2970, the animal chamber may be moved out of the MRI device, and the MR coil is removed.

In 2980, the processing device may determine whether a whole-body scan is complete.

In response to determining that the whole-body scan is not completed, operation 2990 may be performed. In response to determining that the whole-body scan is completed, operation 2995 may be performed.

In 2990, a position of the animal tray within the animal chamber may be moved. After operation 2990 is executed, operations 2930 through 2980 may be repeated.

In 2995, MR images of various body parts of the imaging animal may be stitched to obtain a stitched MR image.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Furthermore, the recited order of processing elements or sequences, or the use of numbers, letters, or other designations thereof, are not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by ±20%. Correspondingly, in some embodiments, the numerical parameters used in the description and claims are approximate values, and the approximate values may be changed according to the required characteristics of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A tray for placing an imaging animal, comprising:
body part fixation portions disposed at fixed positions on the tray and configured to fix body parts of the imaging animal placed on the tray, thereby maintaining a positioning posture of the imaging animal; and
one or more marker accommodation portions configured to accommodate a marker, wherein the marker is detectable in at least one imaging modality.

2. The tray of claim 1, wherein the body part fixation portions comprise:
a head fixation portion configured to fix the head of the imaging animal;
a forelimb fixation portion configured to fix the forelimbs of the imaging animal; and
a hindlimb fixation portion configured to fix the hindlimbs of the imaging animal.

3. The tray of claim 2, wherein each of the forelimb fixation portion and the hindlimb fixation portion includes a groove structure or a hole structure.

4. The tray of claim 2, wherein the head fixation portion includes a bite bar for the teeth of the imaging animal to bite, thereby fixing the teeth of the imaging animal.

5. The tray of claim 2, wherein the body part fixation portions further include a tail fixation portion configured to fix the tail of the imaging animal.

6. The tray of claim 2, wherein:
the forelimb fixation portion includes a first hole structure that penetrates the tray and is configured for the forelimb of the imaging animal to pass through;
the hindlimb fixation portion includes a second hole structure that penetrates the tray and is configured for the hindlimb of the imaging animal to pass through; and
the first hole structure and the second hole structure are waist-shaped holes.

7. The tray of claim 6, wherein:
the imaging animal has a plurality of forelimbs, and the first hole structure includes a first hole structure corresponding to each of the plurality of forelimbs; and
the imaging animal has a plurality of hindlimbs, and the second hole structure includes a second hole structure corresponding to each of the plurality of hindlimbs.

8. The tray of claim 2, wherein the one or more marker accommodation portions include a first marker accommodation portion and a second marker accommodation portion,
the first marker accommodation portion is disposed at a first position on the tray proximate to the forelimb fixation portion, and
the second marker accommodation portion is disposed at a second position on the tray proximate to the hindlimb fixation portion.

9. The tray of claim 1, wherein each marker accommodation portion includes a first accommodation channel, a second accommodation channel, and a marker accommodation cavity,
the first accommodation channel is configured for injecting the marker,
the second accommodation channel is configured for expelling gas during injection of the marker, and
the marker accommodation cavity is respectively in communication with the first accommodation channel and the second accommodation channel and is configured to accommodate the marker.

10. The tray of claim 9, wherein:
the first accommodation channel and the second accommodation channel are hollow cylinders;
the marker accommodation cavity is spherical; and
a center of the marker accommodation cavity is located on a central axis of the first accommodation channel and the second accommodation channel.

11. The tray of claim 9, wherein a diameter of the first accommodation channel is greater than a diameter of the second accommodation channel.

12. The tray of claim 1, wherein the tray is provided with one or more fixing structures, and each marker accommodation portion is detachably mounted to the tray via one of the one or more fixing structures.

13. The tray of claim 12, wherein each fixing structure includes a clamping ring structure, the clamping ring structure includes elastic clamping ring arms configured to secure the corresponding marker accommodation portion.

14. The tray of claim 1, wherein the marker is detectable in at least one first imaging modality, and the marker accommodation portion is detectable in at least one second imaging modality,
the at least one first imaging modality includes at least one of positron emission imaging, single-photon emission imaging, or magnetic resonance imaging (MRI), and
the at least one second imaging modality includes X-ray imaging.

15. The tray of claim 1, wherein a first end portion of the tray for placing the head of the imaging animal is provided with an anesthesia interface configured to deliver anesthetic gas to the imaging animal.

16. The tray of claim 15, wherein the anesthesia interface includes a first anesthesia channel and a second anesthesia channel, the first anesthesia channel including a first end and a second end, the second anesthesia channel including a third end and a fourth end,
the first end is an inlet for the anesthetic gas,
the third end is an outlet for the anesthetic gas and is located near a placement position of the nose of the imaging animal,
the second end is in communication with the fourth end, and at a communication point, a preset angle is formed between the second end and the fourth end.

17. The tray of claim 1, further comprising: a physiological signal acquisition device configured to acquire a physiological signal of the imaging animal.

18. The tray of claim 1, further comprising:
a receiving coil embedded in a bottom of the tray and configured to acquire MR data during an MR scan of the imaging animal.

19. A multi-imaging-animal fixation device, comprising:
a plurality of trays according to claim 1; and
a support portion configured to support the plurality of trays, wherein:
each of the plurality of trays includes a first end portion and a second end portion, the first end portion is configured to place the head of the imaging animal, and the second end portion is disposed opposite to the first end portion;
the support portion includes a first portion and a second portion disposed opposite to each other, the first portion is configured to support the first end portions of the plurality of trays, and the second portion is configured to support the second end portions of the plurality of trays; and
the support portion further includes a third portion configured to connect the first portion and the second portion.

20. The multi-imaging-animal fixation device of claim 19, wherein the first portion is provided with an indication mark for indicating a placement position for the first end portions of the plurality of trays.

21. The multi-imaging-animal fixation device of claim 19, wherein:
the first end portion of each of the plurality of trays is further provided with an anesthesia interface configured to deliver an anesthetic gas to the imaging animal, and the anesthesia interface is provided with an inwardly recessed snap-fit structure;
the first portion of the support portion is provided with a plurality of engagement structures corresponding to the plurality of trays, and each of the plurality of engagement structures includes an outwardly protruding snap-fit boss; and
the snap-fit boss of each of the plurality of engagement structures is configured to engage with the snap-fit structure of the corresponding tray, such that the anesthesia interface and the engagement structure are snap-fit engaged.

22. The multi-imaging-animal fixation device of claim 19, wherein the plurality of trays in the multi-imaging-animal fixation device are arranged in at least two layers, and the trays in different layers are connected via the support portion.

23. The multi-imaging-animal fixation device of claim 19, wherein the first portion, the second portion, and the third portion are independent components capable of being assembled to form the support portion.

24. The multi-imaging-animal fixation device of claim 19, wherein the support portion further includes:
a connector disposed on the first portion and/or the second portion, configured to connect the multi-imaging-animal fixation device and another imaging animal fixation device.

25. An animal chamber, comprising:
a chamber body; and
the tray according to any one of claims 1 to 18, or a multi-imaging-animal fixation device according to any one of claims 19 to 24, wherein the tray or the multi-imaging-animal fixation device is detachably placed within the chamber body.

26. An animal imaging system, comprising:
an imaging device having a scanning cavity; and
the animal chamber according to claim 25, wherein:
the animal chamber is movable into the scanning cavity; and
the imaging device is configured to scan the imaging animal within the animal chamber.

27. The animal imaging system of claim 26, wherein the imaging device includes at least one of a computed tomography (CT) device, an MRI device, a positron emission computed tomography (PET) device, or a single-photon emission computed tomography (SPECT) device, or a multi-modality imaging device formed by a combination of two or more devices selected from the CT device, the MRI device, the PET device, and the SPECT device.

28. A method for scanning an imaging animal, comprising:
scanning the imaging animal using an imaging device to acquire scan data of the imaging animal; and
reconstructing a target medical image of the imaging animal based on the scan data, wherein:
during the scan, the imaging animal is placed on a tray in a preset positioning posture and is placed into the imaging device via the tray; and
the tray includes body part fixation portions disposed at fixed positions on the tray and configured to maintain the preset positioning posture of the imaging animal.

29. The method of claim 28, wherein:
the tray includes an anesthesia interface disposed at a first end portion of the tray, the first end portion is configured for placing the head of the imaging animal; and
during the scan, anesthetic gas is delivered to the imaging animal through the anesthesia interface to keep the imaging animal in an anesthetized state.

30. The method of claim 28, wherein:
the imaging animal includes a plurality of imaging animals, each of the plurality of imaging animals is placed on one tray;
a plurality of trays corresponding to the plurality of imaging animals are placed on a support portion of a multi-imaging-animal fixation device; and
the plurality of imaging animals are placed into the imaging device via the multi-imaging-animal fixation device.

31. The method of claim 30, wherein the scan is a static emission computed tomography (ECT) scan, and before the scan, the method further comprises:
injecting a tracer into the plurality of imaging animals;
after a preset time period following the tracer injection, placing the plurality of imaging animals in an induction box and performing a pre-anesthesia treatment on the plurality of imaging animals; and
placing the plurality of imaging animals after the pre-anesthesia treatment on the plurality of trays in the preset positioning posture.

32. The method of claim 30, wherein the scan is a dynamic ECT scan, and before the scan, the method further comprises:
placing the plurality of imaging animals in an induction box and performing a pre-anesthesia treatment on each of the plurality of imaging animals;
fixing an indwelling needle on each of the plurality of imaging animals after the pre-anesthesia treatment;
placing the plurality of imaging animals after the pre-anesthesia treatment and the fixation of the indwelling needles on the plurality of trays in the preset positioning posture; and
injecting a tracer into each of the plurality of imaging animals using the indwelling needles.

33. The method of claim 28, wherein:
the imaging device includes a first imaging device and a second imaging device, and the scan data includes first scan data acquired by the first imaging device and second scan data acquired by the second imaging device;
the reconstructing a target medical image of the imaging animal based on the scan data includes:
reconstructing, based on the first scan data, a first medical image of the imaging animal;
reconstructing, based on the second scan data, a second medical image of the imaging animal; and
performing registration and fusion on the first medical image and the second medical image to generate the target medical image, wherein the tray is provided with one or more marker accommodation portions configured to accommodate a marker that is detectable in the first medical image and the second medical image, and
the registration and fusion are performed based on the marker in the first medical image and the second medical image.

34. The method of claim 33, wherein one of the first imaging device and the second imaging device is an ECT device, and the other is an MRI device.

35. The method of claim 28, wherein the imaging device is an MRI device, and the scan is an enhanced MR scan; before the scan, the method further comprises:
placing the imaging animal in an induction box and performing a pre-anesthesia treatment and fixing an indwelling needle on the imaging animal;
placing the imaging animal after the pre-anesthesia treatment and the fixation of the indwelling needle on the tray in the preset positioning posture, and placing the imaging animal into the MRI device via the tray; and
performing contrast agent injection on the imaging animal via the indwelling needle.

36. The method of claim 28, wherein the imaging device is an MRI device, and the scan is an enhanced MR scan; the method further comprises:
before the scan:
placing the imaging animal in an induction box and performing a pre-anesthesia treatment and fixing an indwelling needle on the imaging animal;
placing the imaging animal after the pre-anesthesia treatment and the fixation of the indwelling needle on the tray in the preset positioning posture, and placing the imaging animal into the MRI device via the tray; and
connecting the indwelling needle to an external injection device; and
during the scan, performing contrast agent injection on the imaging animal via the external injection device and the indwelling needle.

37. The method of claim 35 or 36, further comprising:
before performing the contrast agent injection on the imaging animal, obtaining an initial MR image by scanning the imaging animal using the MRI device; and
performing comparative analysis on the initial MR image and an enhanced MR image.

38. The method of claim 28, wherein the tray includes a physiological signal acquisition device configured to acquire a physiological signal of the imaging animal; the reconstructing a target medical image of the imaging animal based on the scan data includes:
performing gated reconstruction on the scan data based on the physiological signal to generate the target medical image.

39. The method of claim 30, wherein:
the imaging device is an MRI device;
each tray includes a receiving coil embedded in a bottom of the tray;
the scan data includes MR data acquired by the receiving coil of each tray from the imaging animal placed on that tray; and
the reconstructing a target medical image of the imaging animal based on the scan data includes:
for each tray, generating the target medical image of the imaging animal placed on the tray based on the MR data acquired by the receiving coil of the tray.
